# EUROPEAN PATENT SPECIFICATION

(11) **EP 2 301 450 B1**
(45) Date of publication and mention of the grant of the patent: **23.11.2011**
(21) Application number: 10191770.6
(22) Date of filing: 30.08.2009
(51) Int. Cl.: A61B 17/221

(54) **Embolectomy device**
Embolektomievorrichtung
Dispositif d'embolectomie

(30) Priority: 29.08.2008 US 93173 P; 02.12.2008 US 119369 P
(43) Date of publication of application: 30.03.2011
(62) Divisional of application: 09740999.9
(73) Proprietor: Rapid Medical Ltd., 17906 Shimshit (IL)
(72) Inventor: Eckhouse, Ronen, 17906, Shimshit (IL); Sudin, Yuri, 71700, Modiin (IL); Eckhouse, Shimon, 34980, Haifa (IL)
(74) Representative: Mellet, Valérie Martine

(56) References cited:
- EP-A2- 1 949 921
- WO-A1-2008/057554
- US-A1- 2006 195 137

## Description

### FIELD OF THE INVENTION

The present invention relates to a minimally invasive or surgical device deliverable through an intravascular catheter to the site of an occlusion (e.g., emboli and thrombi). It is designed to remove emboli, even when found in the distal, tortuous neurovasculature. The device includes several sections.

### BACKGROUND OF THE INVENTION

The present invention provides a thrombectomy device which is designed to extract occlusion (e.g., emboli and thrombi) found in the human vasculature, particularly in the distal, tortuous neurovasculature, with a low or minimized pulling or extractive force.

Emboli occasionally form around the valves of the heart and then are dislodged and follow the blood flow into the distal regions of the body. They are particularly dangerous if the clot passes into the neurovascular where it results in an ischemic stroke. As discussed below, many such occlusions occur in the middle cerebral artery (MCA), although it is not the only site where emboli lodge. Obviously, when blood flow is inhibited or cut off completely from a portion of the brain, the brain's oxygen supply is limited causing severe disability or death.

In procedures for removing emboli using the Fogarty catheter or other similar catheters, it is typical, first, to locate the clot using a contrast agent under fluoroscopy. The embolectomy catheter is then inserted and directed to the clot. For example, a distal tip of a balloon catheter may be carefully inserted through the center of the clot. Once the balloon has passed through to the distal side of the clot, the balloon is inflated. The balloon catheter is then gradually and gently withdrawn. The balloon, in this way, acts to pull the clot proximal to the balloon. The majority of procedures using a Fogarty catheter repeat these steps until the pertinent vessel is cleared of clot material.

Such vaso-occlusions occur in a wide variety of sites within the body. The lodging of clots in various sites is complicated by the presence of atherosclerosis. This disease causes the vessels to become tortuous and narrowed. These anomalies are often considered to be the result of the growth of atherosclerotic plaque. Clots occurring in these diseased vessels are difficult to remove using balloon or Fogarty catheters.

Removal of emboli using balloon catheters is rife with potential problems. One such problem occurs during removal of a clot. The resistance to such removal often causes the balloon portion of the catheter to evert over the tip of the catheter. Should the user need to partially deflate the balloon during such a deflation, the distal tip of the balloon may become distended and angulated. Another difficulty with balloon catheters is the possibility of damage to the intima of arteries. Inflation pressures can create forces significant enough to shear such a vessel lining or to dislodge plaque lodged on such a wall. In the worst case, the balloon may rupture leaving balloon detritus in the bloodstream.

Movement of a balloon in the MCA can displace the clot through more proximal branches into other large vessels such as the internal carotid artery (ICA) and then into other vessels

There are a variety of different devices intended for use in replacing balloon catheters and in using a device other than a balloon catheter in so removing the emboli

One such device is shown in U.S. Pat. No. 4,030,503 to Clark III. This patent describes a spiral helix affixed to the distal end of a catheter. In particular, the spiral helix is designed to be rotated and pushed forward through the clot. It is said that the helix screws into the clot, and when it is firmly embedded or is past the clot, the catheter is pulled out of the vessel without rotation. The catheter is said to operate like a corkscrew.

A similar catheter is described in U.S. Pat. No. 4,706,671 to Weinrib. This catheter also has a coil section at its distal end. The coil section is said to be stretched initially into a generally linear insertion position for removing inwardly in a vessel. The coil member is then expanded into the form of a hollow conical scoop to then scoop clot material from the blood vessel. The coil member is stiffened by an internal wire which is then removed. The hollow passageway is then filled with a liquid to stiffen the coils. The coils are said to be of an elastomeric material.

U.S. Pat. No. 4,762,130 to Fogarty et al., describes a helical balloon attached to the distal end of a catheter. The helical or bellowed balloon is maintained in a generally linear condition and passed into a clot. Once the catheter balloon within the clot is inflated, the balloon and adjoining clot are removed together.

Another similar device used more to grip and shear atherosclerotic deposits rather than to remove thrombi is described in U.S. Pat. No. 4,890,611 to Monfort et al. This device incorporates a pair of helical wires placed on the distal end of a wire. The flexible wire is pulled against a flexible catheter, the helical loops cut through and is said to retain sections of plaque for removal.

A thrombus extraction system is shown in U.S. Pat. No. 5,011,488, to Ginsberg. In this device, a deflated (but inflatable) balloon having a proximal conic shape is passed through a targeted thrombus. The balloon is then expanded and retracted so that the proximal end pulls the thrombus into contact with an aspirator. The aspirator then removes the thrombotic material from the vessel.

The Ginsburg patent describes an alternative wire-based configuration of the expandable member. In this variation, a wire coil is attached to an extension wire which may be moved between an extended position and a retracted position. The retracted or expanded configuration is illustrated to have a conical shape. The cone's proximal end is shown to be smaller than the distal end.

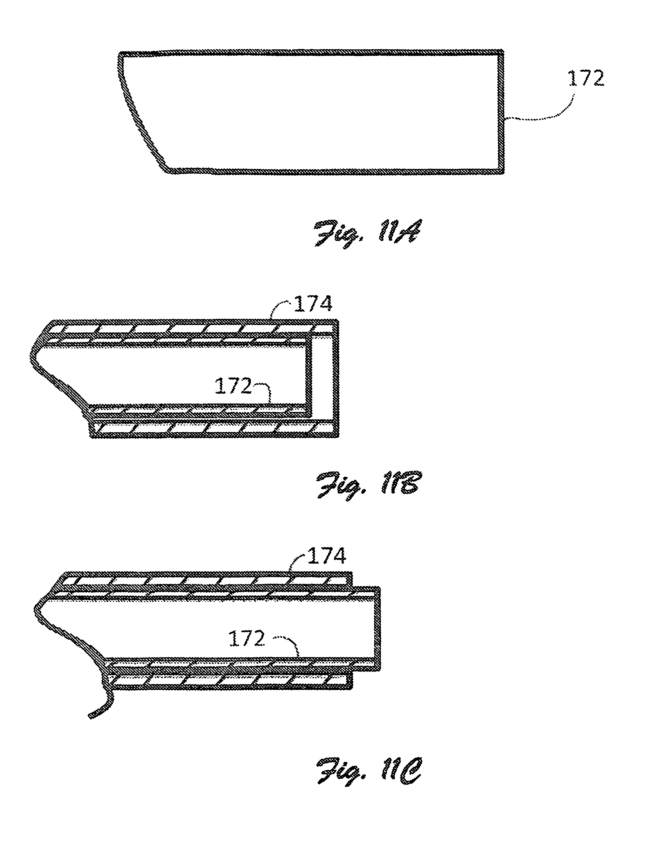

U.S. Pat. No. 5,112,347, to Taheri, shows an inflatable balloon-type embolectomy catheter. The balloon has a number of "fingers" arranged in a leaf spring arrangement inside the balloon. The balloon is hydraulically inflated and forms a cone after inflation. The deflated device is shown in FIGS. 11 through 14 to be passed distally past a clot before inflation. After inflation, the large end of the balloon collects the embolism as it is pulled past the appropriate site in the vessel.

U.S. Pat. No. 5,193,386, to Phan et al., shows a retrieval catheter for removing materials from various body lumens. The retrieval catheter is shown to have a slack net which may be collapsed for passage into lumen past the material to be collected. The net is unfolded after such passage and materials such as uretral stones are removed.

U.S. Pat. No. 5,411,509, to Hilal, shows an embolectomy catheter having a distal elastomeric foam tip. The foam tip has an actuator means suitable for forming the foam section both longitudinally and radially in response to activation of the actuation. In practice, the catheter tip is pressed past an embolism, inflated, and retracted proximally along with the clot.

U.S. Pat. No. 5,490,859, to Mische et al., shows an intravascular occlusion material removal device having an expandable material removal element made up of a number of wires passing between the two ends of such element, a catheter shaft, a drive shaft for spinning the element within the blood vessel, and a collection portion placed on the material removal element for collecting any occlusion material removed by the expandable material removal element. The drive shaft may be operated by a motor connected to the drive shaft proximate to the proximal end of the drive shaft.

Another patent literature relates to the use of a net which encircle the clot. Such teaching can be found in US application no. US2005/0101986. US application no. US20060287670, US application no. US20070038241, US application no. US20030009189 and PCT application on. WO9923976. The major drawback of such nets are the fact that the clot or clot might fragments within the blood vessel and hence not be extracted as a whole from said blood vessel.

US application no. US2006195137 to Sepetka relates to devices and methods for removing an obstruction from a blood vessel are described. The devices are deployed in a collapsed condition and are then expanded within the body. The devices are then manipulated to engage and remove the obstruction.

US application no. US2006195137 claims a method of removing an obstruction from a blood vessel, comprising the steps of: providing an obstruction removing element having an elongate element, the elongate element forming a plurality of expandable structures along an obstruction engaging portion, the plurality of structures being movable from a collapsed position to an expanded position, the plurality of structures being formed by the elongate element with adjacent structures being formed so that adjacent structures are wound in opposite directions; positioning the obstruction removing element in the collapsed position; advancing the obstruction removing element into an obstruction; and permitting the obstruction engaging element to expand after the advancing step so that the plurality of structures move toward the expanded position and engage the obstruction.

EP application no. EP1949921 to Microvention Inc. relates to embolectomy catheters, rapid exchange microcatheters, systems and methods for removing obstructive matter (e.g., thrombus, thromboemboli, embolic fragments of atherschlerotic plaque, foreign objects, etc.) from blood vessels. This invention is particularly useable for percutaneous removal of thromboemboli or other obstructive matter from small blood vessels of the brain, during an evolving stroke or period of cerebral ischemia. In some embodiments, the embolectomy catheters of this invention are advanceable over a guidewire which has been pre-inserted through or around the obstructive matter. Also, in some embodiments, the embolectomy catheters include obstructive matter capturing receptacles which are deployable from the catheter after the catheter has been advanced at least partially through the obstructive matter; Such obstructive matter capturing receptacles may include i) at least one proximal strut which are designed to be retractable through a blood clot and ii) a distal matter-receiving portion which is designed to prevent a blood clot from passing therethrough. In particular, the embolectomy catheter may be a rapid exchange embodiment.

PCT publication no. WO2008057554 to Cook Inc. relates to a thrombus removal device includes a shaft with a distal end and a proximal end, a sheath with a distal end and a proximal end, and a helical coil attached at a proximal end to the distal end of the shaft and is disposed within the lumen of the sheath in a closed configuration. The helical coil includes a plurality of body portions with turns spaced apart longitudinally and laterally to facilitate screwing the helical coil into a thrombus and also providing an open area into which the thrombus can be captured. A distal tip of the helical coil is provided with a loop, an angle of which is about the same as the angle of at least one body portion. The helical coil assumes an open configuration when the sheath is retracted proximally from the distal tip. An aspiration device may be provided in fluid communication with the center of the helical coil.

None of above mentioned patents and patent applications discloses a device which is especially adapted to apply radial compression forces and longitudinal shearing forces on the clot so as to mitigate the tendency of the clot to fragment.

### SUMMARY OF THE INVENTION

The present invention provides a minimally invasive medical device deliverable through an intravascular catheter to the site of a occlusion. It is designed to remove clot from the vasculature, and is particularly useful in removing clot from distal, tortuous vasculature.
The device of the present invention is designed to remove clot, even when found in the distal, tortuous neurovasculature.
The present invention provides an embolectomy device for removal of clot from vasculature comprises a rotatable spring-like helical member having a plurality of loops and a tubular mesh-like net.
The rotating member may be configured to expand to approximate the diameter of the vessel and to at least partially envelope the clot and also to aid in the separation of the clot from the vessel wall.
The rotating member approximate the clot and envelopes it by its rotation. The rotating member may also be used with various intravascular catheters or especially design ones, as will be described hereinafter.
The rotating member may serve several purposes. First, the loops of the rotating element are configured so that it will separate the clot from the arterial wall, at least partially envelope the clot, manipulate the clot reciprocally and/or rotationally with respect to the main longitudinal axis of the clot and extracts it.
The mesh-like net encapsulates the clot (confined within the coil) therein.
It is one object of the present invention to provide a device for extracting a clot from a blood vessel in a non fragmented manner, comprising:
an endless wire coupled to a spring-like helical member having a plurality of loops; said loops are positioned at an angle A relatively to the main longitudinal axis of said blood vessel, and, are adapted to radially encircle at least a portion of the outer circumference of said clot; and,
a tubular mesh-like net for both enveloping said helical member and enclosing said clot therein;
wherein at least a portion of said clot is contemporaneously confined within said loops and said mesh-like net, such that radial compression forces and longitudinal shearing forces are exerted on said clot and the tendency of said clot to fragment is mitigated.
It is another object of the present invention to provide the embolectomy device as defined above, wherein said spring-like helical member and/or said mesh-like net comprising means for applying vibration on said clot.
It is another object of the present invention to provide the embolectomy device as defined above, wherein said mesh-like net is made of material selected from a group consisting of woven fabrics, non-woven fabrics, gauze-like fabrics, materials having multiple openings, and fluid-permeable membranes or any combination thereof.
It is another object of the present invention to provide the embolectomy device as defined above, wherein said mesh-like net additionally comprising materials selected from a group consisting of woven fabrics, non-woven fabrics, gauze-like fabrics, materials having multiple openings, and fluid-permeable membranes or any combination thereof.
It is another object of the present invention to provide the embolectomy device as defined above, wherein said spring-like helical member is coupled to said mesh-like net.
It is another object of the present invention to provide the embolectomy device as defined above, wherein said distal portion of said mesh-like net is adapted to switch from an expended-configuration to a restricted-configuration; said expended-configuration is operative to allow insertion of at least a portion of a target clot in to said mesh-like net's distal portion; and said restricted configuration is operative to retain said clot within said mesh-like net.
It is another object of the present invention to provide the embolectomy device as defined above, wherein said distal portion of said mesh-like net is operative to self-expand from its expended-configuration to its restricted-configuration.
It is another object of the present invention to provide the embolectomy device as defined above, wherein said mesh-like net is an elongated sleeve comprising a restricting sphincter located at the distal end of said sleeve, adapted to retain at least a portion of the target clot within said mesh-like net.
It is another object of the present invention to provide the embolectomy device as defined above, wherein said helical member is characterized by a cross-sectional area selected from a group consisting of circular, oval, round, square, rectangular, triangular, regular or irregular shapes, or any combination thereof.
It is another object of the present invention to provide the embolectomy device as defined above, additionally comprising a motor adapted to rotate said helical member.
It is another object of the present invention to provide the embolectomy device as defined above, wherein said helical member additionally comprising protrusions for assisting in the separation of said clot from said vasculature.
It is another object of the present invention to provide the embolectomy device as defined above, additionally comprising vibration means applied on said clot.
It is another object of the present invention to provide the embolectomy device as defined above, additionally comprising a corkscrew-like effecter, having a coil-like distal end, adapted to penetrate and cross said clot.
It is another object of the present invention to provide the embolectomy device as defined above, wherein the diameter of said helical member is variable so as to better adjust to the diameter of said clot or said blood vessel.
It is another object of the present invention to provide the embolectomy device as defined above, wherein said device additionally comprising:
a proximal effecter characterized by a folded configuration and an open configuration;
a distal effecter characterized by a folded configuration and an open configuration;
said proximal effecter, in said open configuration is adapted for grasping a proximal portion of said clot; said proximal effecter, in said open configuration is adapted for grasping a distal portion of said clot;
wherein both said effecters, when open, are oppositely positioned and are adapted to operate in concert, for trapping said clot such that said clot is (i) manipulatable along and/or around the main longitudinal axis of said blood vessel; and, (ii) extracted out of said blood vessel.
It is another object of the present invention to provide the embolectomy device as defined above, wherein said distal effecter is operative to cross said clot in said FOLDED configuration and then to be expanded to an OPEN configuration.
It is another object of the present invention to provide the embolectomy device as defined above, wherein said distal effecter is operative to self-expand from its FOLDED configuration to its OPEN configuration.
It is another object of the present invention to provide the embolectomy device as defined above, wherein said distal effecter is incorporated or otherwise connected with said proximal effecter to form a single effecter.
It is another object of the present invention to provide the embolectomy device as defined above, wherein said angle A ranges from about 0 degrees to about 180 degrees.
It is another object of the present invention to provide the embolectomy device as defined above, additionally comprising aspiration means.
It is another object of the present invention to provide the embolectomy device as defined above, wherein at least one of said loops and/or said mesh-like net is coated with lubricious polymeric material selected from hydrophilic polymer material.
It is another object of the present invention to provide the embolectomy device as defined above, wherein said spring-like helical member is characterized by single, double, or triple pitch.
It is another object of the present invention to provide the embolectomy device as defined above, wherein the cross section of at least one of said loops and/or said mesh-like net varies.

### BRIEF DESCRIPTION OF THE DRAWINGS

Figure 1a illustrates an out-of-scale manner a schematic view (cross section) of the device according to a preferred embodiment of the present invention.
Figures 1b-1g illustrating an out-of-scale manner a schematic view (cross section) partially illustrating the minimally invasive embolectomy device according to one embodiment of the invention (namely the coil).
Figures 2a to 2e illustrate the entire device (i.e., the coil and the tubular mesh-like net) as described by the present invention.
Figure 3a illustrates a second embodiment of the device as described by the present invention in which an inner corkscrew-like effecter is utilized.
Figures 4a-4c illustrating the rotatable spring-like helical effecter integrated with the tubular mesh-like net.
Figures 5a-5k illustrate an embodiment in which the device additionally comprising a distal and proximal clasps.
Figure 6 is a view of an intravascular catheter according to the present invention and an associated guide wire.
Figures 7A, 7B, 7C, illustrate another embodiment of the catheter provided by the present invention.
Figures 8A and 8B illustrate another embodiment of the catheter provided by the present invention.
Figures 9A and 9B illustrate another embodiment of the catheter provided by the present invention.
Figure 10A illustrates the rotatable spring-like helical effecter of the embolectomy device protruding out of the catheter.
Figures 11A, 11B, and 11C illustrate another embodiment of the catheter provided by the present invention.
Figure 12 illustrates another embodiment of the catheter provided by the present invention.
Figure 13 illustrates another embodiment of the present invention.
Figures 14A and 14B illustrate the operation of a heat activated variation of the catheter.
Figure 15 illustrates a schematic depiction of a methods of using the catheter according to one embodiment of the present invention.
Figures 16A and 16B illustrate the embolectomy device utilizing the rotatable spring-like helical effecter.
Figure 16C illustrates a variation of a rotating helical assembly.
Figures 17A and 17B illustrate another embodiment of the embolectomy device utilizing the rotatable spring-like helical effecter.
Figure 17C illustrates another variation of the rotatable spring-like helical effecter.
Figures 18A-18C illustrate the distal tip of the catheter with a rotating element configured to remove a clot as a whole from an artery.
Figure 19 shows a procedure for removing a clot using the variation shown in Figures 18A-18C.
Figures 20A, 20B(1), and 20B(2) show further variations of the device in which the catheter has an expandable distal-most portion.
Figure 21 shows a partial cutaway side view of one variation of the expanded catheter tip.
Figure 22 shows a perspective view of the device shown on Figure 20B(2).
Figures 23A and 23B show a partial cutaway side view of the expanded catheter tip.
Figures 24 and 25 show perspective views of other variations of the expanded catheter tip.
Figure 26 shows a method for using the device depicted in Figures 16A and following.

### DETAILED DESCRIPTION OF THE PREFERRED EMBODIMENTS

The following description is provided, alongside all chapters of the present invention, to enable any person skilled in the art to make use of said invention and sets forth the best modes contemplated by the inventor of carrying out this invention. Various modifications, however, will remain apparent to those skilled in the art, since the generic principles of the present invention have been defined specifically to provide a minimally invasive implement that is designed to restore flow in human vasculature by extraction of clots from the same, even in distal, narrow neurovasculature.

The term "**crossing**" refers hereinafter to act of intersecting clot and penetrating its interior.

The term "**about**" refers hereinafter to a range of 25% below or above the referred value.

The term "**non-fragmented manner**" refers hereinafter to extraction of clots intact. i.e., the clot is removed as a whole without the danger of fragmenting within the vasculature.
The device of the present invention is intended to be used as a flow restoration device. Even in those instances where the clot is not or cannot be completely removed, the device of the present invention is believed to be useful in removing the clot in a non-fragmented manner (i.e., as a whole) and thereby permitting restoration of partial blood flow.
Furthermore, the device of the present invention is sufficiently flexible to be placed in distal tortuous anatomy and hence is useful in treating blocking clot found there.

As described, the present invention provides an embolectomy device for extracting a clot in an intact manner from a blood vessel without fragmenting it. The device comprises an endless wire having a distal end. The distal end comprising a spring-like helical member having a plurality of loops. The loops are positioned at an angle A relatively to the main longitudinal axis of said blood vessel, and radially encircle [without crossing] at least a portion of the outer circumference of the clot. It is acknowledge that angle A ranges from about 0 degrees to about 180 degrees, namely 90 to 180 degrees.

The device also comprises a tubular mesh-like net for both enveloping the helical member and enclosing the clot therein.

By configuring the embolectomy device to contain both the mesh-like net and the spring-like helical member - coil, the entire clot mass is confined within the mesh-like net during reciprocal movement (i.e., extraction) of the clot, so as to provide *(i)* extraction of the entire mass of the clot from the blood vessel (in a non-fragmented manner); and, *(ii)* prevention of re-entry of clot mass or fragments thereof into the blood vessel.

Further more, by contemporaneous confining the clot within the loops and the mesh-like net, radial compression forces and longitudinal shearing forces are exerted on said clot such that the tendency of said clot to fragment is mitigated.

Treatments for embolic occlusions include catheterization of the patient and introduction of tissue plasminogen activator (t-PA), urokinase, or streptokinase to the site of the occlusion. Additionally the embolic occlusion may be penetrated-often with a microcatheter-and the t-PA or urokinase or streptokinase introduced distally of the occlusion.

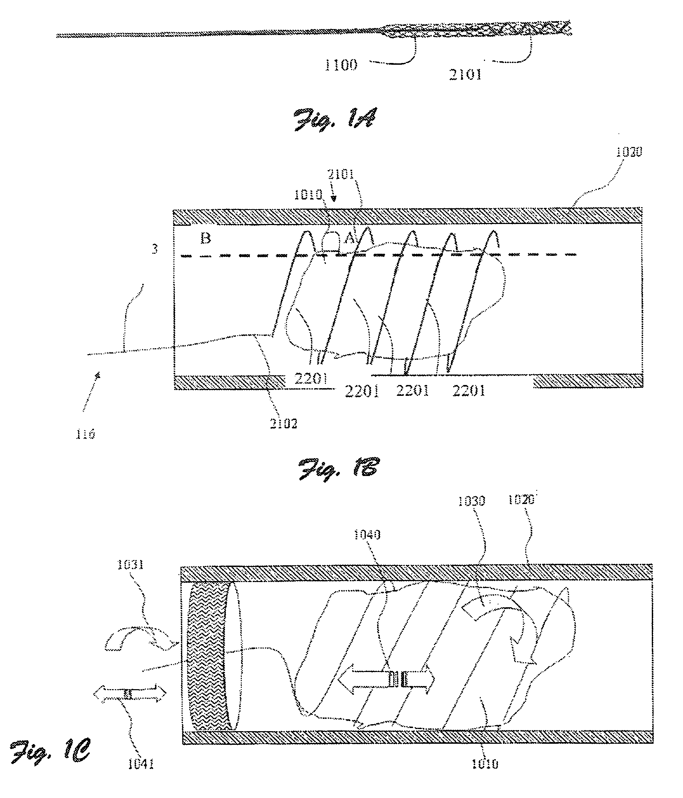

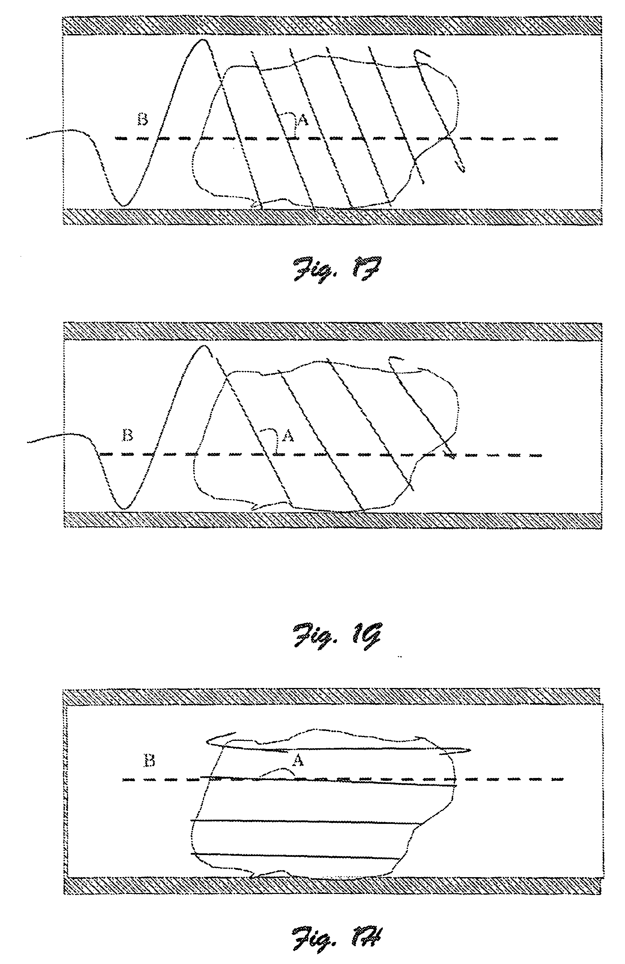

Reference is mow made to figure 1a illustrating an out-of-scale manner a schematic view of the device as provided by the present invention. As can be seen from the figure, the device comprises a rotatable spring-like helical member 2101 and a tubular mesh-like net 1100.

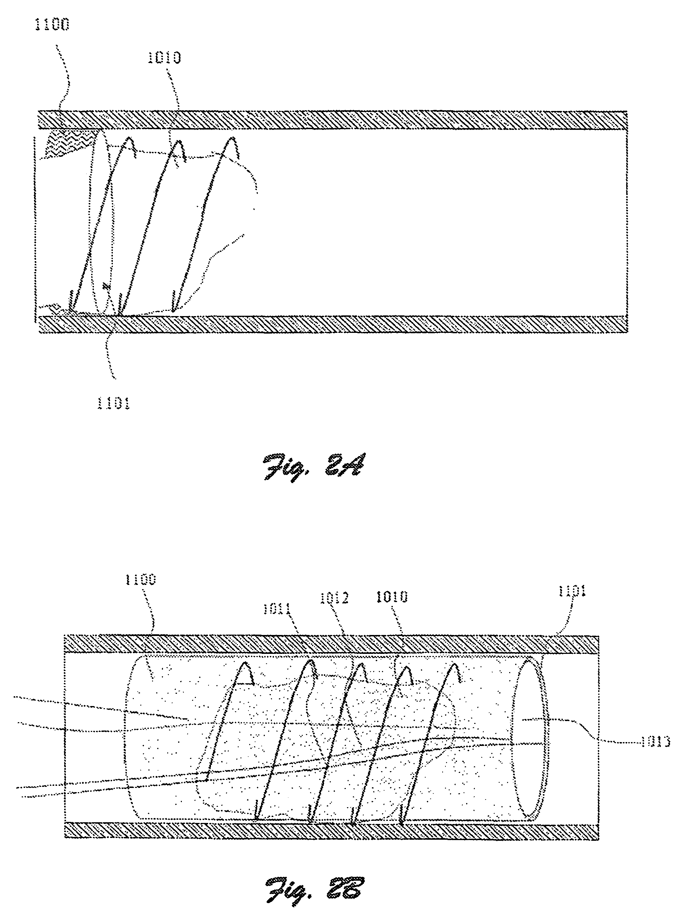

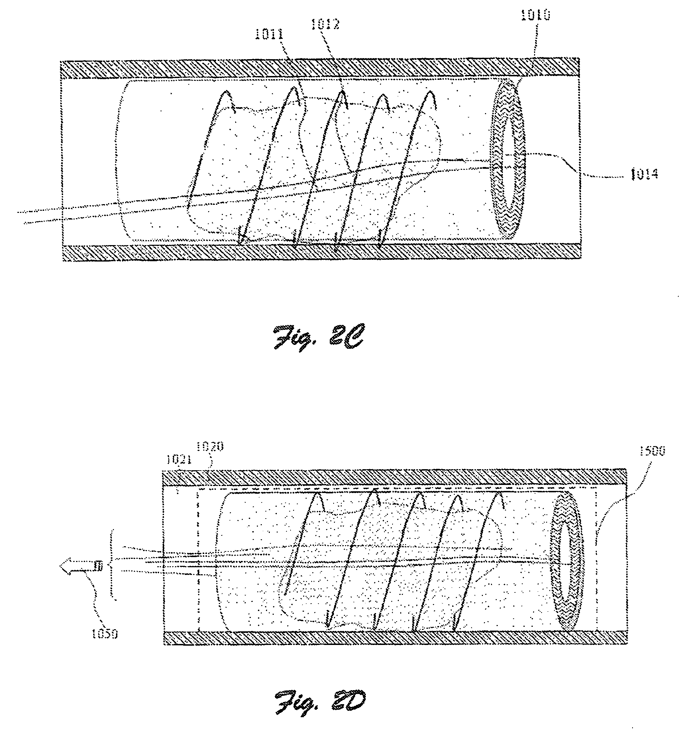

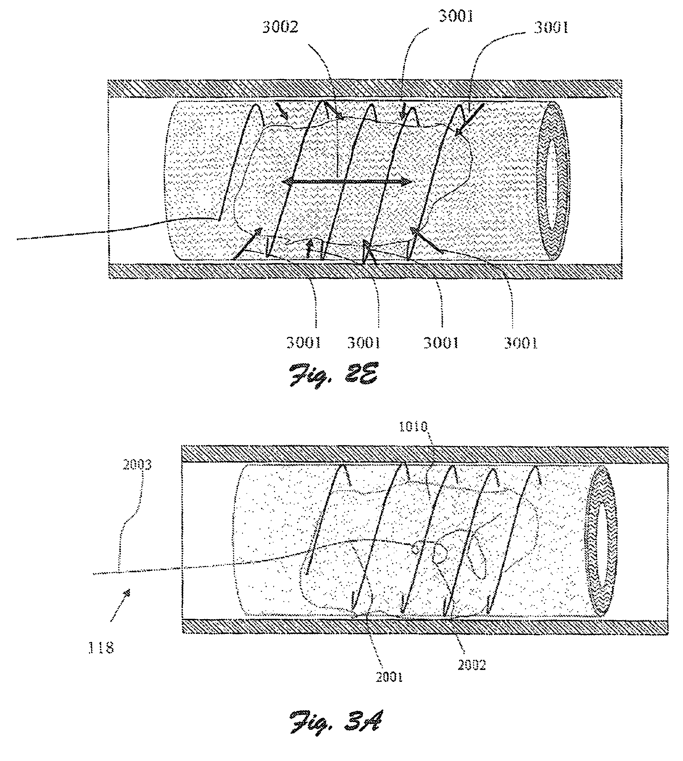

Figures 1b-1d presenting in an out-of-scale manner a schematic view (cross section) of the rotatable spring-like helical member 2101 used in the minimally invasive embolectomy device according to one embodiment of the invention (the tubular mesh-like net will be described in figures 2).

The device, according to one embodiment, comprises a rotatable spring-like helical member, adapted to grasp and separate the clot from the artery wall and extracts it from outside of the artery. The helical member comprises a plurality of loops which are positioned at an angle *A* relatively to the main longitudinal axis of said blood vessel. It is acknowledged that angle *A* ranges from about 0 degrees to about 180 degrees, namely 90 to 180 degrees.

Said grasping and separating is performed by spiral-like encircling (i.e., enveloping, without crossing) of at least a portion of the outer circumference of the clot by the plurality of loops. The encircling is performed without intersecting or crossing the clot; and by manipulating the clot reciprocally and/or rotationally with respect to the main longitudinal axis.

Such a feature of extracting a clot without crossing it is highly desired for enabling an extraction in an intact manner of the clot (i.e., extraction of the clot's entire mass; in a non fragmented manner).

Crumbling of the clot may occur when the clot is crossed (i.e., intersected), and fragments can be left within the blood vessel.

The device additionally comprises a tubular mesh-like net (as illustrated in the following figures, namely figure 2) for both enveloping the helical member and enclosing the clot therein. The mesh-like net enables extraction of the entire clot mass during the extraction in a non fragmented manner, such that reentry of clot mass or fragments thereof into said blood vessel is prevented.

The mesh-like net (as will be described hereinafter) may have several features, e.g., mechanical, chemical, thermal, etc., allowing it to engage a clot or embolism. The mesh-like net may also be in communication with a vacuum source allowing enhanced grasp of the clot or embolism for removal or other treatment. The mesh-like net may be used with a guidewire to direct the catheter to the site of the clot or clot.

In light of that, the shaft of the mesh-like net is hollow so as to enable introduction of wires.

According to some embodiments, the spring-like helical member may include one or more distally located, protrusions for assisting in the separation of the clot or embolism from the wall of the artery.

According to other embodiment, the spring-like helical member and/or the mesh-like net may comprise means for applying vibration on the clot so as to facilitate its extraction.

As a matter of practical experience, a large proportion of emboli sloughed into the brain are seldom longer than about 1.5 centimeters and, for a variety of reasons both physiological and hemodynamic, often settle in the middle cerebral artery (MCA). The path from the site of origin of a traveling clot --often the heart-- to the MCA is via the common carotid artery past the branch point forming the external and internal carotid arteries into the internal carotid artery (ICA). The MCA is generally considered to be the continuation and termination of the ICA past the siphon and pat the branching of a variety of other arteries, e.g., the ophthalmic artery, the anterior communicating artery, , and others.

The etiology of such an occlusion is varied, varying, and complicated. Treatments for such embolic occlusions include catheterization of the patient and introduction of tissue plasminogen activator (t-PA), urokinase, or streptokinase to the site of the occlusion. Additionally the embolic occlusion may be penetrated or crossed.

As described above, the device of the present invention is intended to be used in removing clots or at least recanalizing (at least partially) occluded vascular lumen.

Reference is made again to figure 1b presenting in an out-of-scale manner a schematic view (cross section) of a minimally invasive embolectomy device according to one embodiment of the invention.

We will first illustrate the grasping of the clot by means of the wire having a plurality of loops and then the extraction of the grasped clot by the mesh-like net.

As described above, the device is designed to extract clot (1010) found in the human vasculature (1020), particularly in the distal, tortuous neurovasculature with a low or minimized pulling or extractive force outside the body of the patient.

The device comprises an endless wire (116) having a distal end (2102). The distal end comprising a spring-like helical member (3101) for capturing the clot (1010). The helical member comprising a plurality of loops (2201) positioned at an angle A relatively to the main longitudinal axis (as illustrated in the figure as B) of the blood vessel (1020).

The loops radially encircling at least a portion of the outer circumference of the clot (1010). It is acknowledged that angle A ranges from about 0 degrees to about 180 degrees, namely 90 to 180 degrees.

It should be emphasized that the plurality of loops (2201) effectively separates the clot from its outer surface *without* crossing the clot.

Hence, the loops are configured to (i) spirally encircle (e.g., envelope) the clot without intersecting and crossing the clot; and, (ii) manipulate said clot reciprocally and/or rotationally with respect to the main longitudinal axis of the clot; such that its separation from the internal wall of the vessel is facilitated.

The loops warp the outer circumference of the clot, and apply a preset and well directed force, to grasp the clot in a vector which is approximately parallel to its main longitudinal axis, namely a force applied from and towards the clot's distal and/or proximal poles.

Reference is thus made to figure 1c which illustrates the manipulation that can be exerted on the clot by the loops. The endless wire, as described above, is characterized by a distal spool (spring-like) portion. By applying rotational maneuvers and/or linear reciprocating movement (1031 and 1041, respectively), clot 1010 reciprocated and/or circularly actuated (1040 and 1030, respectively), until it is separated from vascular wall 1020.

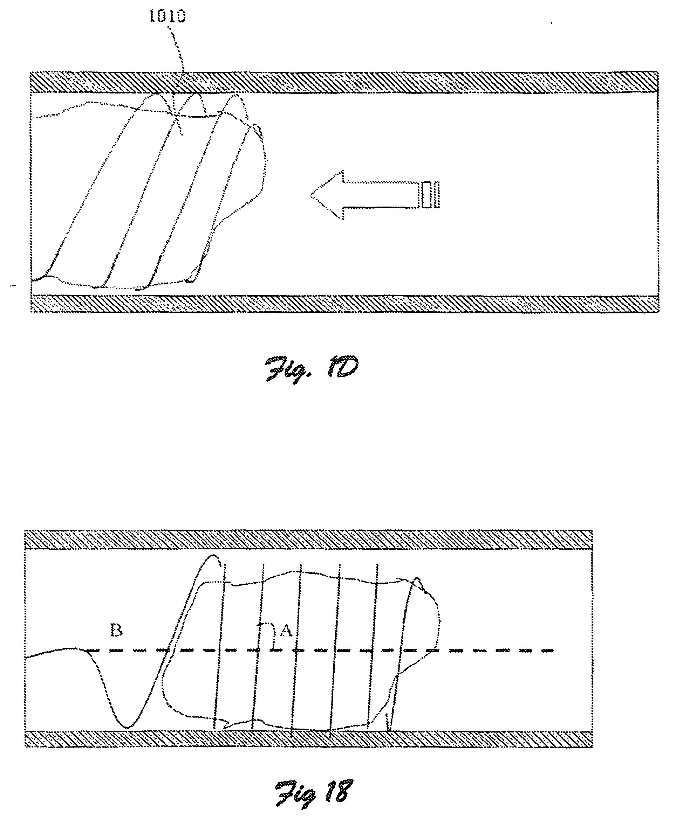

Consequently, as illustrated in figure 1d, working wire is pulling the clot into a standard catheter, which in return extracts it from outside of the body.

As mentioned above, the loops are positioned at an angle A relatively to the main longitudinal axis of the blood vessel (1020) and angle A ranges from about 0 degrees to about 180 degrees, namely 90 to 180 degrees.

Figures 1e-1h illustrate a clot encircled by said plurality of loops at different angles.

According to one embodiment, the coil tips that can be utilized is selected from a group consisting of a loop at the end, a bulge or any combination thereof.

As described above, the device also comprises a tubular mesh-like net which is an essential member of the device. The mesh-like net will be described hereinafter.

Reference is now made to figure 2a which illustrates the tubular mesh-like net 1100.

According to one embodiment of the present invention, once the clot (1010) has been separated from the vascular wall and encircled by the loops, it is inserted within the tubular mesh-like net (1100) via its distal inlet (1101), as see figure 2a. therefore, the clot is first confined by the loops and only then encircled by the mesh-like tubular net.

According to another embodiment, the clot is simultaneously encircled by the loops and entrapped by the mesh-like net, according to yet another embodiment, the clot is first entrapped by the mesh-like net and only then it is encircled by the loops.

In each of the above described embodiments the device is adapted to *(i)* reversibly envelope and grasp clot (1010) (via the loops and the mesh-like net) and, in doing so, separates the clot from the vascular wall; and then, *(ii)* when clot (1010) is secured within mesh-like net (1100) and the loops, extracting the enveloped clot form within the body outside the body.

Hence, the device is useful to (i) free the clot from its surrounding vascular wall by applying reciprocal/rotational maneuvers; (ii) enforce the clot towards the mesh-like net's distal inlet in a safe manner, i.e., in a way the clot does not break to small pieces and does not escape, i.e., as a whole; and (iii), position the clot, as is, within the mesh-like net in a safe manner, i.e., in a way in which the clot does not disengage to small pieces and does not escape, as a whole, downstream away from the mesh-like net.

It is emphasized that when the clot is contemporaneously confined within the loops and the mesh-like net, radial compression forces and longitudinal shearing forces are exerted on the clot, such that the tendency of said clot to fragment mitigates.

Reference is now made to figure 2b illustrating different embodiments of the mesh-like net. According to one embodiment of the invention, the distal inlet of the mesh-like net (1101) is a constricting sphincter-like orifice. The diameter of orifice is regulated, e.g., by means of operating wires 101 and 1012, by electrical charge (in case of Nitinol made sphincters) by electro-active polymers, by temperature (e.g., heat in case of Nitinol made sphincters) etc.

By pulling wires 1011 and 1012, or by activating the distal portion of the mesh-like net 1010, the diameter of the orifice is reduced, e.g., from open configuration 1013 to semi close configuration 1014 (see figure 2c), or to a totally close configuration (not shown here).

According to another embodiment of the invention, all operating wires are pulled (1050) and clasped clot 1500, now free from its native vascular wall 1020 is pulled via predetermined track 1021 outside of the patent body (see figure 2d).

Reference is now made to figure 2e illustrating the key innovation of the present invention. According to the present invention, two combined forces are applied on said clot once said clot is contemporaneous confined within the loops and the mesh-like net:

(a) radial compression forces applied by the mesh-like net 3001; and,

(b) longitudinal shearing forces applied by the loops 3002. As described above, the loops warp the outer circumference of the clot, and apply a preset and well directed force, to grasp the clot in a vector which is approximately parallel to its main longitudinal axis, namely a force applied from and towards the clot's distal and/or proximal poles.

The combination of said two forces exerted on the clot mitigates tendency of the clot to fragment, such that it can be extracted from the blood vessel wholly (i.e., its entire mass is extracted).

It should be emphasized that the force is applied locally, i.e., no stress is applied on adjunctive vessels.

When one pulls or compress an object, a plurality of tearing points will be created within said object. The main feature of the present invention lies in the fact that the combination of the two forces mitigates any tearing that might occur as a result of massive compression or pulling forces.

It is according to yet another embodiment of the invention wherein an inner-coil assembly is utilized.

Reference is now made to figure 3a, in which the device additionally comprising a corkscrew-like effecter 118, having a coil-like 2002 distal end, adapted to effectively anchor the clot from its inner portion, and an endless wire 2001 which terminates in a operating portion 2003 of the effecter, locate in the very proximal end, namely outside the body of the patient.

It is in the scope of the invention wherein the corkscrew-like effecter is utilized either with or without the rotatable spring-like helical effecter.

It is acknowledge that sequence of grabbing and extracting the clot via the mesh-like net, the loops (of the spring-like helical member), and the corkscrew-like effecter can be varied.

According to another embodiment of the present invention each of the above described loops can additionally comprise vibration means can be utilized to assist in removing the clot from the vascular wall.

According to another embodiment, aspiration can be applied to assist in the separation and extraction of the clot from the vessel.

According to another embodiment, the endless wire and/or the spring-like helical member and/or the mesh-like net are coated with lubricious polymeric material such as a hydrophilic polymer material, e.g., one containing polyvinylpyrrolidone.

According to another embodiment, the endless wire and/or the spring-like helical member and/or the mesh-like net may have multiple polymeric layers and include woven braids, flat ribbon coils, or wire coils between those layers to provide stiffness, kink resistance, etc.

According to another embodiment, the endless wire and/or the spring-like helical member and/or the mesh-like net are characterized by a varied diameter so as to better fit the clot.

According to another embodiment, the endless wire and/or the spring-like helical member and/or the mesh-like net are characterized by several different diameters along its length so as to better fit the clot

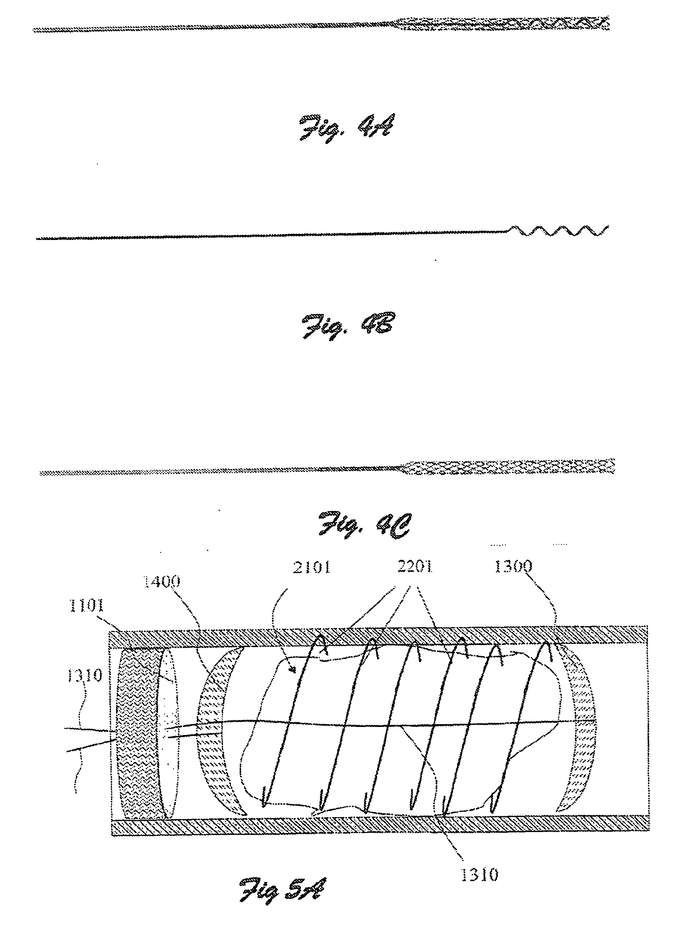

Reference is now made to figures 4a-4c illustrating the wire and loops integrated with the tubular mesh-like net (see figure 4a); solely the wire and loops (see figure 4b); and, solely the tubular mesh-like net (see figure 4c).

It is acknowledge that there are numerous operating modes of the device:

**First mode**

(a) the clot is approximated by the integrated spring-like helical member and mesh-like net;

(b) the spring-like helical member protrudes out of the distal end of the device;

(c) the spring-like helical member is rotated such that at least a portion of the outer circumference of the clot is encircled by the plurality of loops and confined within the same;

(d)the spring-like helical member confining at least a portion of the clot is pulled and inserted into the tubular mesh-like net; and,

(e) the mesh-like net is extracted from the blood vessel in a non fragmented manner (i.e., the entire mass of the clot is extracted);

(f) the device together with the clot are removed from the body.

**Second mode**

(a) the clot is approximated by the integrated spring-like helical member and tubular mesh-like net;

(b) the spring-like helical member is rotated to approximate the clot whilst simultaneously the tubular mesh-like net approximates the same;

(c) as a result of such rotation, at least a portion of the outer circumference of the clot is encircled by the plurality of loops and confined within the loops and the mesh-like net; and,

(d) the mesh-like net confining the loops (which encircles the clot) is extracted from the blood vessel in a non fragmented manner (i.e., the entire mass of the clot is extracted).

**Third mode**

(a) the clot is approximated by the integrated spring-like helical member and mesh-like net;

(b) the tubular mesh-like net protrudes out of the distal end of the device and enclose the clot;

(c) the spring-like helical member protrudes out of the distal end of the device;

(d) the spring-like helical member is rotated such that at least a portion of the outer circumference of the clot is encircled by the plurality of loops and confined within the same; and,

(e) the mesh-like net is extracted from the blood vessel in a non fragmented manner (i.e., the entire mass of the clot is extracted).

Additionally, as described above, the clot can be crossed via the corkscrew-like effecter 118.

Reference is now made to another embodiment of the present invention, according to which the device additionally comprising between two opposing, multi-element collection assemblies, i.e., distal clasp and proximal clasp. The two clasps combined with the spring-like helical member will extract the clot with a low or minimized pulling or extractive force in an intact manner (i.e., the entire clot is extracted from the body).

Reference is now made to figures 5a-5k illustrating said embodiment combining a distal and proximal clasp together with the spring-like helical member.

Figure 5a illustrates the spring-like helical member (2101) and the plurality of loops (2201). which are positioned at an angle *A* relatively to the main longitudinal axis of the blood vessel 1020. It is acknowledged that angle *A* ranges from about 0 degrees to about 180 degrees.

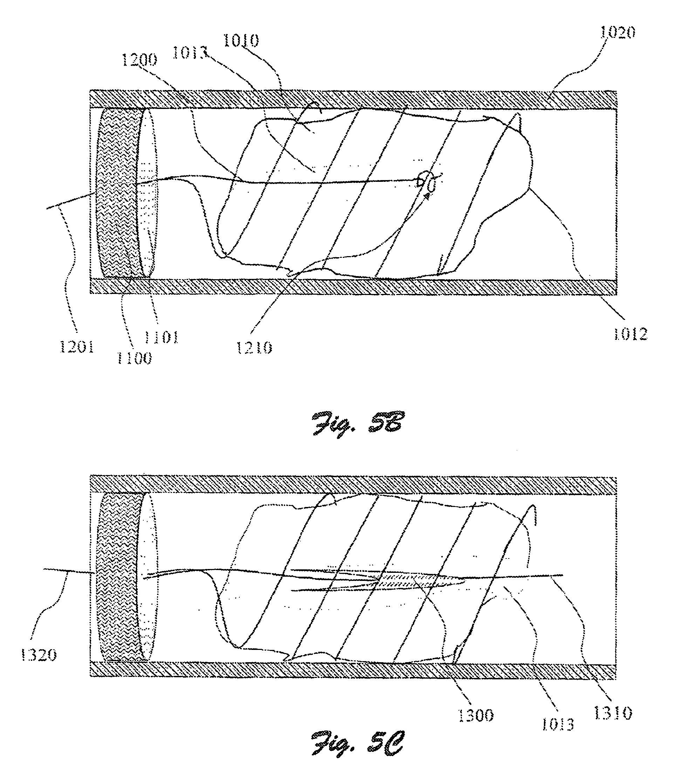

The combined device is adapted to *(i)* reversibly grasp a clot (1010) between two opposing, multi-element collection assemblies, i.e., distal clasp (1300) and proximal clasp (1400) and, confined within the plurality of loops. In doing so, the clot is separated from the vascular wall. The device is also adapted to *(ii)* insert the clot (1010) within of mesh-like net (1100, see figure 5b) via it's the distal inlet (1101); and then, *(iii)*, when clot (1010) is secured within mesh-like net (1100), extracting enveloped clot form within the body.

The following disclosure discloses the operation of the clasps. It should be emphasized that the claps can be activated prior to, during or after the clot has been grasped by the spring-like helical member (2101).

The following figures demonstrate the activation of the claps after the clot has been encapsulated via the loops (2201) of the spring-like helical member (2101). However, as mentioned above the clot can be grasped by the claps prior to or simultaneously being encapsulated by the loops.

Figure 5b illustrates a clot (1010) in an occluded vascular lumen (1020). A mesh-like net-like graft (1100) is inserted within lumen (1020), e.g., in a percutaneous manner, such that its distal end is located adjacent the clot. Guidewire 1200, operated front its proximal portion (1201), is inserted through the mesh-like net (1100) and manipulated to cross the clot, e.g., by means of an effecter 1210 located in its very distal end. The effecter is selected, in a nonlimiting manner, from a group consisting of a coil-like, screw-like or drill-like head, reciprocally and/or rotatably operated in one or by a sequence of maneuvers.

According to one possible embodiment of the invention, a small-diameter bore (1013) is provided within clot (1010). According to yet another embodiment, guidewire (1200) crosses the clot without forming a noticeable bore.

Figure 5c illustrates yet another embodiment of the invention, wherein distal clasp (1300) is reversibly folded to a thin arrow-like member, crossing the clot by means of a guidewire. According to this embodiment, distal clasp (1300) has a FOLDED-configuration, e.g., an arrow-like spiral wound or otherwise collapsed member of a respectively thin cross section and narrow circumference; and an OPEN-configuration, e.g., a canopy, cymbal or mesh-like net-like member of a wider cross section and longer circumference. The endless guidewire 1013 is operated in its proximal portion (1320) outside the body of patient, and exceeds to the clot's distal end (1310).

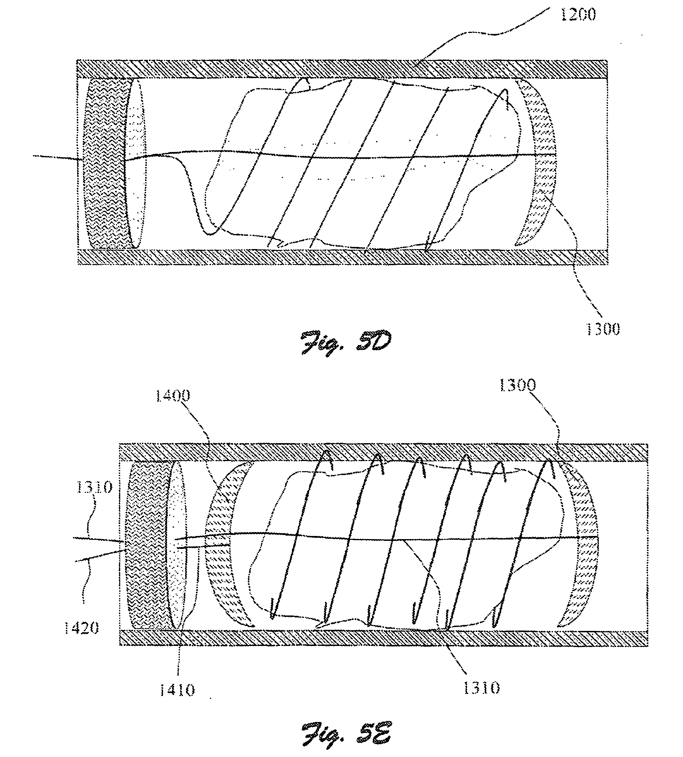

Figure 5d illustrates the distal clasp (1300) in its OPEN configuration. The diameter of the clasp, in this illustration, is adapted to fit in size and shape the inner diameter of the blood vessel 1020. Figure 5e illustrates both distal clasp (1300) and proximal clasp (1400) in their OPEN configuration. Distal clasp and proximal clasp are manipulated by means of at least one operating wires 1310 and 1410, respectively.

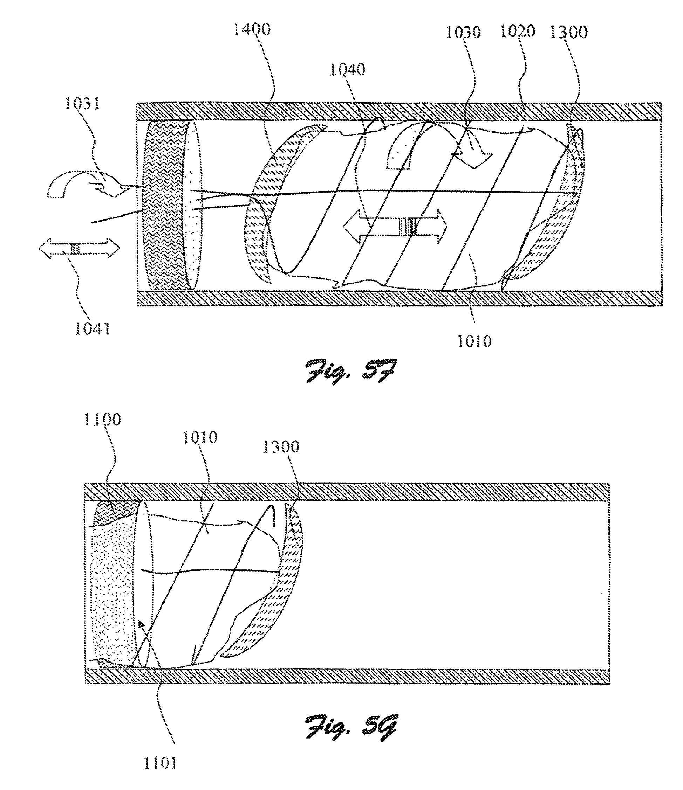

Figure 5f illustrates a possible mode of action of the device especially adapted to *(i)* reversibly grasp clot (1010) between the two opposing distal clasp (1300) and proximal clasp (1400) and confined within the loops of the spring-like helical member.

One or both of the clasps are manipulated, e.g., either linearly (1041) or rotatably (1031) by means of operating wires in, e.g., *(i)* a single reciprocal stroke along the long longitudinal axis of the blood vessel, wherein clot is maneuvered in a reciprocate facilitated motion (1040); *(ii)* a set of reciprocal motions, for example in-and-out opposite motions, a vibrating set of reciprocal motions, etc.; *(iii)* a single rotating stroke around the long longitudinal axis of the blood vessel, wherein clot is maneuvered in a circle facilitated motion (1030); *(iv)* a set of rotational motions, for example circular opposite motions, a vibrating set of circular motions, etc.; and (*v*) any combination of the same. By those maneuvers, the clot is separated from the vascular wall 1020.

Figure 5g illustrates the step wherein clot (1010) which was separated from the vascular wall is inserted within mesh-like net (1100) via its distal inlet (1101), by pulling distal clasp (1300) inside the mesh-like net.

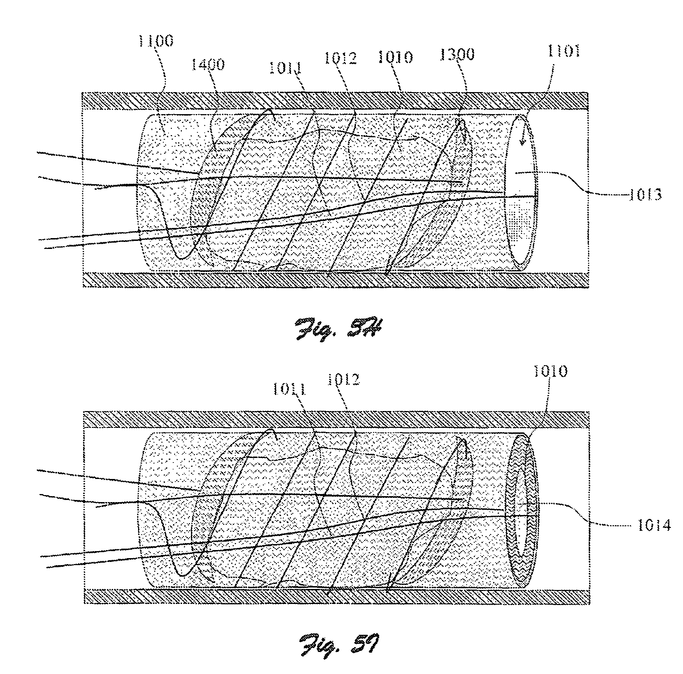

Figures 5h and 5i illustrate each a secured physical conjugate of an enveloping mesh-like net 1100, containing distal and proximal clasps (1300 and 1400, respectively) and the loops of the spring-like helical member that immobilize clot 1010 within the structure.

According to annother embodiment of the invention, distal inlet of the mesh-like net (1101) is a constricting sphincter-like orifice. The diameter of orifice is regulated, e.g., by means of operating wires 1011 and 1012, by electrical charge (in case of Nitinol made sphincters) by electro-active polymers etc. By pulling wires 1011 and 1012, or by activating the distal portion of the mesh-like net 1010, the diameter of the orifice is reduced, e.g., from open configuration 1013 to semi close configuration 1014, or to a totally close configuration (not shown here).

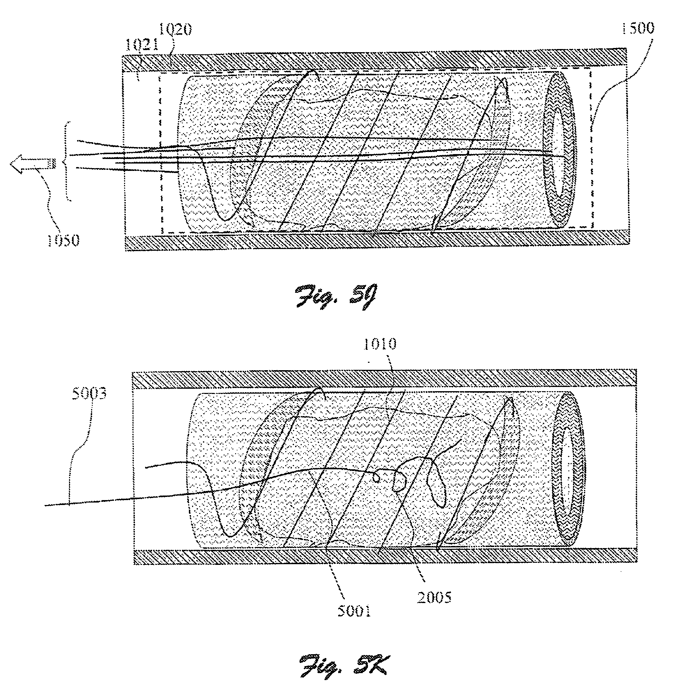

Figure 5j illustrates the last step, in which the enveloped clot (see conjugate 1500) is safely extracted form within the body outside the body. According to another embodiment of the invention, all operating wires are pulled (1050) and clasped clot 1500, now free from its native vascular wall 1020 is pulled via predetermined track 1021 outside of the patient's body.

It is according to yet another embodiment of the invention wherein an inner-coil assembly is utilized. Reference is now made to figure 5k, presenting a corkscrew-like effecter (as described above), having a coil-like distal end (5002), adapted to effectively anchor the clot from its inner portion, and an endless wire (5001) which is terminated in a operating portion of the effecter, locate in the very proximal end (5003), namely outside the body of the patient.

It is acknowledged in this respect that distal and proximal clasps as defined in any of the above are provided useful to effectively clasp a clot from its both distal and proximal surfaces in an opposite cymbal-stroking coordinated manner and thus to *(i)* free the clot from its surrounding vascular wall by applying reciprocal/rotational maneuvers; *(ii)* enforce the clot towards the mesh-like net's distal inlet in a safe manner, i.e., in a way the clot does not disengage to small pieces and does not escape, as a whole, downstream away from the mesh-like net; and *(iii),* positioned the clot, as is, within the mesh-like net and providing a proximal-distal structural clot-grasping element in a safe manner, i.e., in a way the clot does not disengage to small pieces and does not escape, as a whole, downstream away from the mesh-like net.

The claps combined with the plurality of loops of the spring-like helical member further provide a better grasping of the clot so as to provide a full extraction.

It is acknowledged that each and both the corkscrew-like effecter implanted within the clot, and the spring-like helical member warping the outer circumference of the same, are adapted to apply a preset and well directed force, and to grasp the clot in a vector which is approximately parallel to its main longitudinal axis, namely a force applied from and towards the clot's distal and/or proximal poles. Hence, both internal and external coils are provided useful to (i) free the clot from its surrounding vascular wall by applying reciprocal/rotational maneuvers; (ii) enforce the clot towards the mesh-like net's distal inlet in a safe manner, i.e., in a way the clot does not disengage to small pieces and does not escape, as a whole, downstream away from the mesh-like net; and (iii), positioned the clot, as is, within the mesh-like net and providing a proximal-distal structural clot-grasping element in a safe manner, i.e., in a way the clot does not disengage to small pieces and does not escape, as a whole, downstream away from the mesh-like net.

It should be emphasized that the combined device (i.e., containing both the spring-like helical member having a plurality of loops and the distal and proximal claps) may operate by different modes of operations (i) first encircling the clot by the loops and mesh-like net; then grasping the same by the claps; (ii) first grasping the clot by the claps and then encircling the same via the loops and mesh-like net; or, (iii) simultaneously grasping the clot by the claps and encircling the same via the loops and mesh-like net.

It should be pointed out that both the spring-like helical member (and hence the loops) and the tubular mesh-like net are characterized by elastic mechanical properties which enables them to contract and hence to apply pressure on the clot.

According to another embodiment of the present invention, an especially dedicated catheter is provided. Said dedicated catheter which will be discussed hereinafter. This catheter system comprises several important sections that may be used cooperatively together with the above mentioned tubular mesh-like net and/or the above mentioned spring-like helical member. Alternatively it may be provided as a stand alone device.

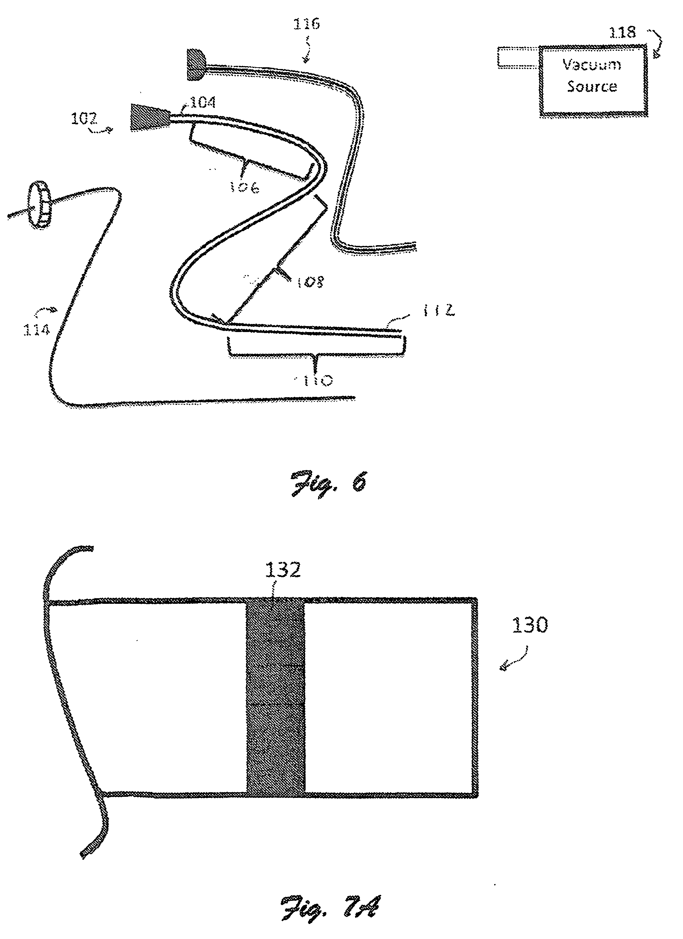

Reference is now made to figure 6 illustrating an intravascular catheter (102) that may have the distal clot-engaging features.

Also shown in the figure a guidewire (114) that may be used to direct the intravascular catheter (102) to the target site. Typically, such an intravascular catheter (102), when used in the neurovasculature, is a microcatheter that may be made up of a number of sections (104, 106, 108, 110) of different flexibilities.

The more-distal sections are typically more flexible and often have decreasing diameter. The most distal section (110) of a suitable microcatheter may be, for instance, 2.0-5.0F. 2.5-3.5F. The distal end of the catheter (102) often includes a radio-opaque marker (112).

The catheters may be produced from a variety of materials. Catheters produced by integrating layers of different compositions to achieve specific results are also well known. For instance, providing a thin inner layer of a lubricious polymer (including fluoropolymers such as polytetrafluoroethylene (PTFE or TFE), ethylene-chlorofluoroethylene (ECTFE), fluorinated ethylene propylene (FEP), polychlorotrifluoroethylene (PCTFE), polyvinylfluoride (PVF), or polyvinylidenefluoride (PVDF)) to allow easy movement of guidewires is appropriate. Other appropriate inner liner materials include polyethylene, polypropylene, polyvinyl chloride (PVC), ethyl vinyl acetate (EVA), polyurethanes, polyamides, polyethylene terephthalate (PET), and their mixtures and copolymers. Outer coverings of polyethylene or of EVA or their mixtures, copolymers, etc. or thermoplastic elastomers, including those containing polyesters as components, such as HYTREL are useful.

A variety of other polymers may be used, depending upon the use to which the catheter section is placed. For instance, if the section is a proximal section, stiffer polymers such as polyimides, polyamides (such as the Nylons), high density polyethylene (HDPE), polypropylene, polyvinylchloride, various fluorocarbon polymers (for instance: PTFE, FEP, vinylidene fluoride, their mixtures, alloys, copolymers, block copolymers, etc.), polysulfones, or the like, are appropriate. Blends, alloys, mixtures, copolymers and block copolymers of these materials are also suitable if desired.

If a more flexible section is required, those sections may comprise a more flexible material such as polyurethanes, low density polyethylene (LDPE), polyvinylchloride, THV, etc. and other polymers of suitable softness or a modulus of elasticity.

Each of the polymers noted herein may be used in conjunction with radio-opaque filler materials such as barium sulfate, bismuth trioxide, bismuth carbonate, powdered tungsten, powdered tantalum, or the like so that the location of various portions of the catheter sections may be radiographically visualized within the human body.

The catheters may have multiple polymeric layers and include woven braids, flat ribbon coils, or wire coils between those layers to provide stiffness, kink resistance, etc.

The catheter may be covered or coated with a lubricious polymeric material such as a hydrophilic polymer material, e.g., one containing polyvinylpyrrolidone.

An endless wire (116) is coupled to a spring-like helical member having a plurality of loops is used.

A suitable guidewire (114) may be used to deliver the distal end of the catheter (102) to a site proximate to the clot. If the endless wire (116) is not used, the guidewire (114) is then removed from the catheter (102) and the vacuum source (118) attached to aspirate the clot.

The endless wire (116) may be used with intravascular catheter (102) having distal features for engaging a clot or a catheter not having those features.

In any case, the spring-like helical member, namely the loops, of the endless wire (116) may be inserted in the catheter and rotated to draw the clot inside the catheter.

Figure 7A shows a close-up, side-view of the distal end (130) of one variation of the catheter, (102). A radio-opaque marker band (132) is also shown.

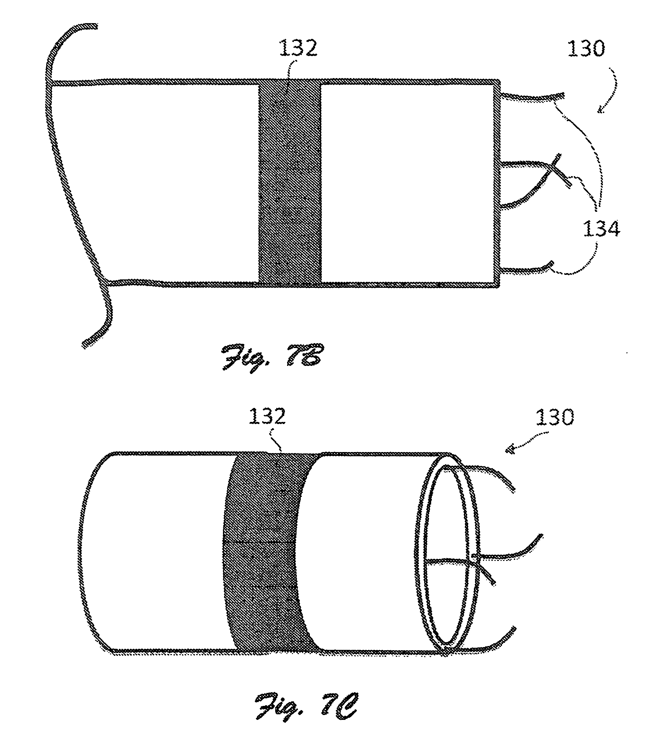

Reference is now made to figure 7B illustrating another embodiment, according to which, a set of hooks (134) are extended from the distal end of the catheter when its in a position adjacent a clot. Said hooks are provided in order to engage with the clot, e.g., by penetrating the clot and hence alleviate its release from the vessel.

It should be mentioned that a vacuum may be applied through the catheter lumen.

Figure 7C shows a perspective view of the distal end (130) showing the hooks (134). In this view, all the hooks (134) rotate in the same direction as they extend from that distal end. The direction of the extended hooks (134) may be varied to enhance the strength of the clot's grasping.

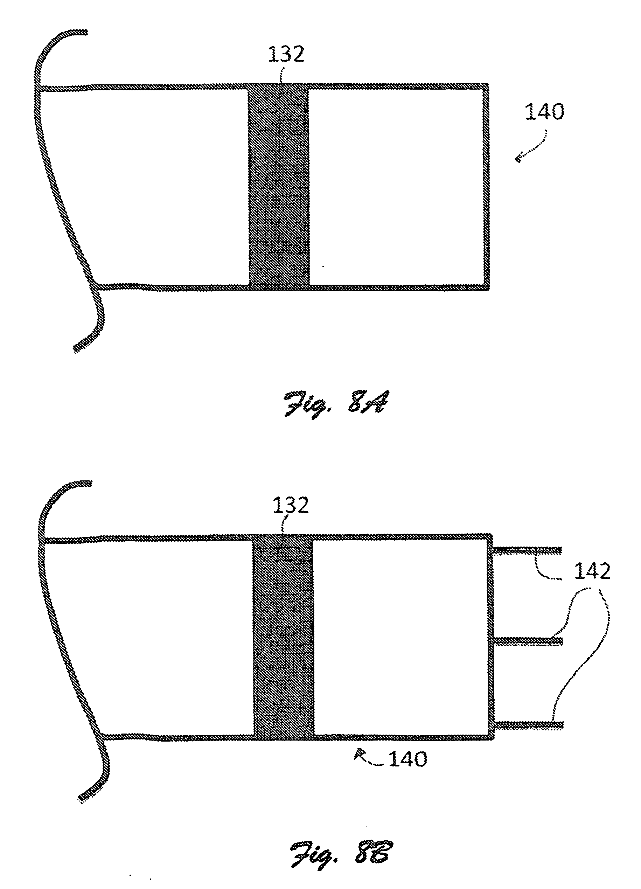

Figure 8A shows a close-up, side-view of the distal end (140) of another variation of the catheter component (102). A radio-opaque marker band (132) is also shown. When this variation is introduced to a position adjacent to the clot, a set of pins (142) is extended from that distal end (see Figure 8B) to engage the clot, e.g., by penetrating the clot.

It should be mentioned that a vacuum may be applied through the catheter lumen for facilitating the extraction of the clot.

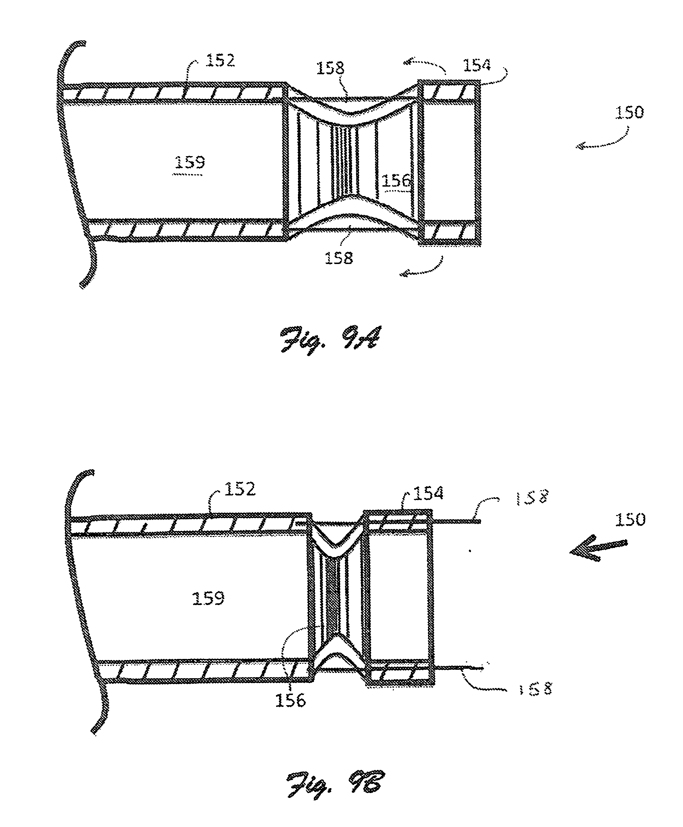

Reference is now made to figures 9A and 9B illustrating the distal end of a catheter having a collapsible structure that collapses upon imposition of a vacuum and extends hooks (134 in Figure 8B) or pins (142 in Figure 8B) into a clot.

Figure 9A shows a close-up, cross-sectional, side-view of the distal end of the catheter component (150). The catheter body (152) extends proximally (as shown in Figure 6). A movable distal ring (154) is separated from the catheter body (152) by a collapsible bellows (156). The bellows (156) are shown in the "at rest" (fig. 9B) or extended position (fig. 9A). After the catheter contacts the clot, the catheter lumen is essentially sealed. Subsequent imposition of a vacuum upon that catheter lumen causes (i) the bellows (152) to collapse; (ii) the distal ring (154) to slide proximally, and the pins (158) to extend distally into the clot.

Figure 9B shows the bellows (152) in a collapse condition and the pins (158) extending through the distal ring (154) into the clot.

As a matter of general design criteria, the bellows (156) should be sufficiently stiff so that during passage of the catheter tip to the clot site (and the inevitable contact of that tip with the catheter walls), the bellows (156) will not collapse allowing extension of the pins (158) during that transit. Conversely, the bellows (156) should be just stiff enough that when the distal end of the catheter contacts the clot and a vacuum applied to the catheter lumen (159), the bellows (156) will collapse.

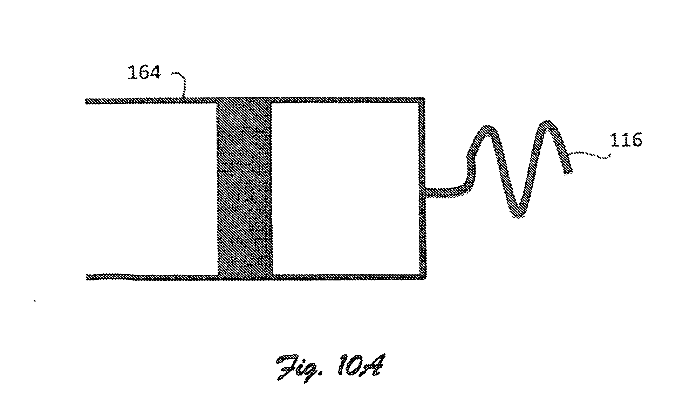

According to an embodiment of the present invention, the combination of the catheter, as any of the described above, utilizing the endless wire having a spring-like helical member which comprises a plurality of loops (see figure 10A, which illustrated the loops extending from the catheter body) is provided.

Reference is now made to figure 11A illustrating another embodiment of the catheter as provided by the present invention. In said embodiment, the active clot's engaging feature is a reactive or adhesive region (172) that adheres to a targeted clot upon contact with that clot.

Figures 11B and 11C show the combination of the catheter shown in Figure 11A with an exterior slideable sleeve (174) that, in Figure 11B, is extended to protect the reactive or adhesive region (172) as the catheter passes to the target clot. Figure 11C shows the sleeve (174) after retraction to expose the reactive or adhesive region (172).

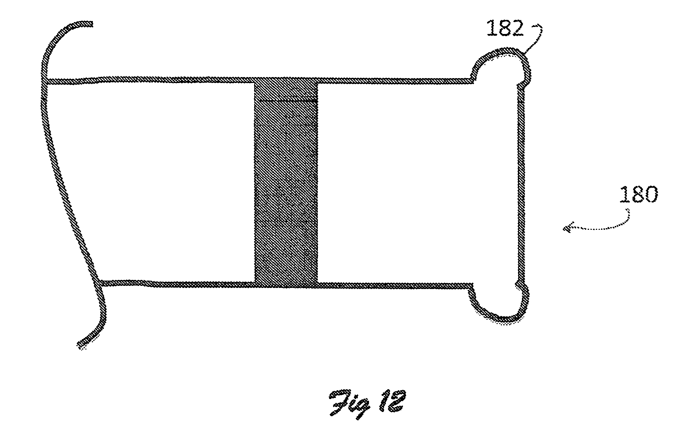

The catheter (180) shown in Figure 12 includes a nosepiece (182) containing a heating element that either directly heats the clot upon contact with the clot or activates a grasping end on the catheter upon heating.

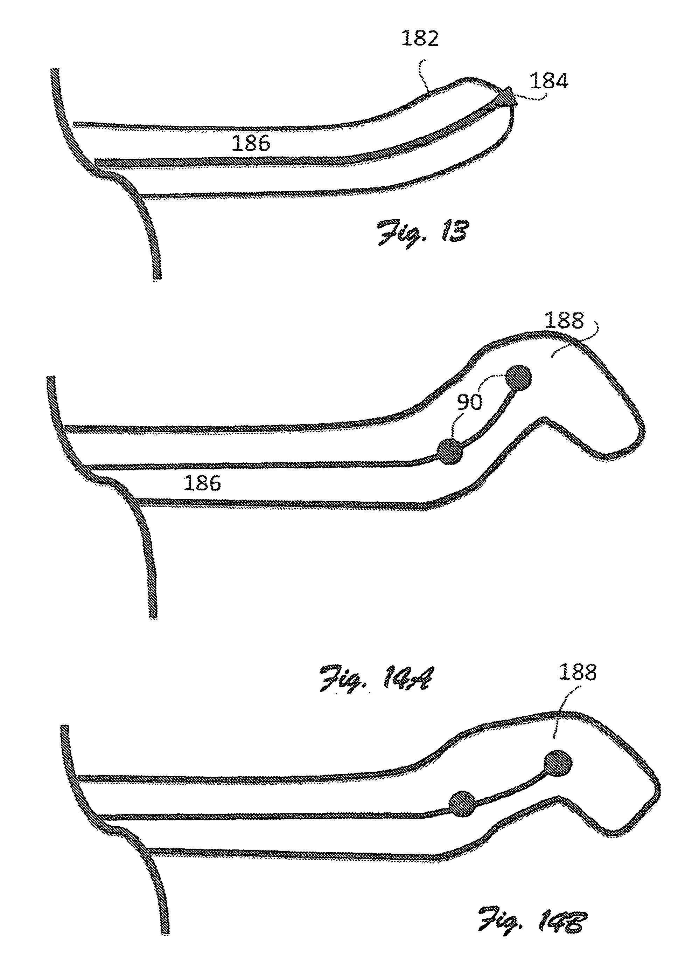

Figure 13 illustrates a partial, cross-sectional, side-view of the distal tip (182) of the catheter component (180) shown in Figure 12. The heating element (184) in this variation is placed distally to provide maximum heat transfer to the clot. Electrical current is supplied through wire (186).

Figures 14A and 14B illustrate a variation of the catheter component (180) found in Figure 12 having a heat-actuated region that shrinks upon that heating to grasp the clot.

Figure 14A provides a partial cross-sectional view of the distal tip of the variation shown in Figure 12. The catheter body (186) comprises a distal tip (188) having a heat-shrinkable polymer, e.g., pre-oriented polyolefin, polyvinylchloride (PVC), fluoropolymers such as FEP, PTFE. polyvinylidene fluoride (e.g., KYNAR), neoprene, silicone elastomers, and fluorocarbon elastomers (e.g., VITON, FLUOREL). The distal tip (188) contains one or more heating elements (190) which, when heated, cause that distal tip (188) to shrink and grasp the clot and perhaps shrink the clot itself. In Figure 14B, the distal tip (188) has shrunk to provide a smaller opening.

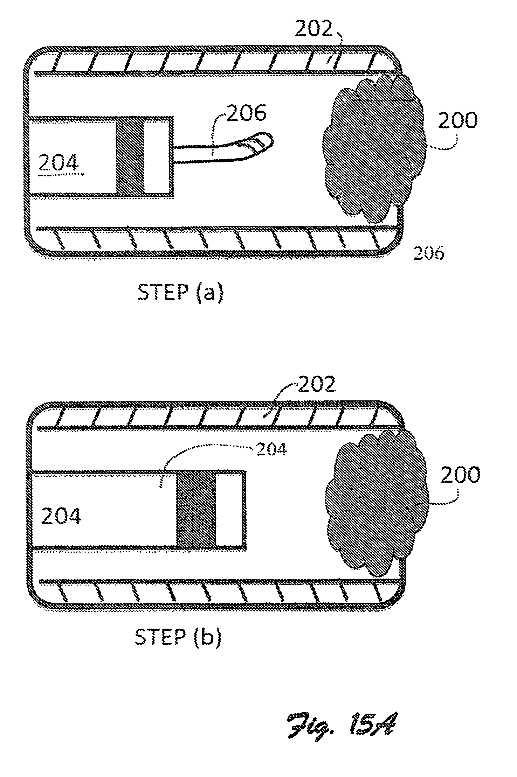

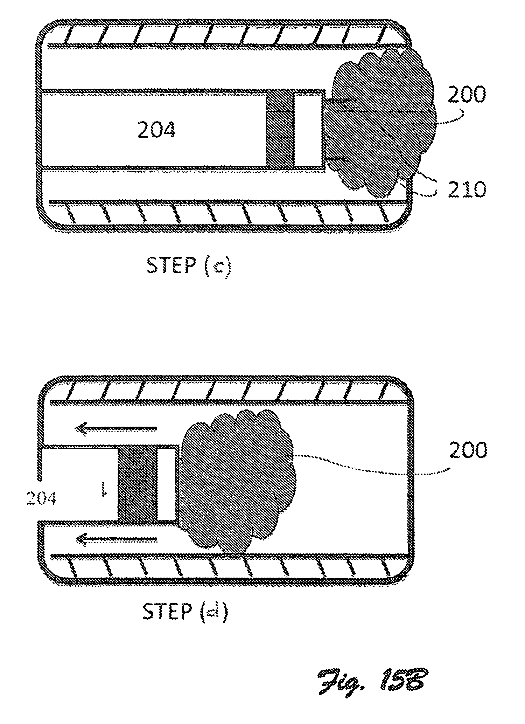

Figure 15 schematically shows a typical procedure for using one of the different variations of the catheter as described above (i.e., the extending pins) in order to remove a clot (200) from an artery (202).

In step (a), the distal end of a microcatheter (204) has been placed in the vicinity of the target clot (200) using a guidewire (206).

In step (b), the microcatheter (204) has approached the clot (200). The guidewire (206) that was used to direct that microcatheter (204) has been withdrawn.

In step (c), the microcatheter (204) has contacted the clot (200). A clot engaging componentin this example: extending pins (210) -- has been extended into the clot and the catheter (204) connected to a vacuum source to secure the catheter (204) to the clot (200).

In step (d), the catheter (204) is being withdrawn along with the clot (200).

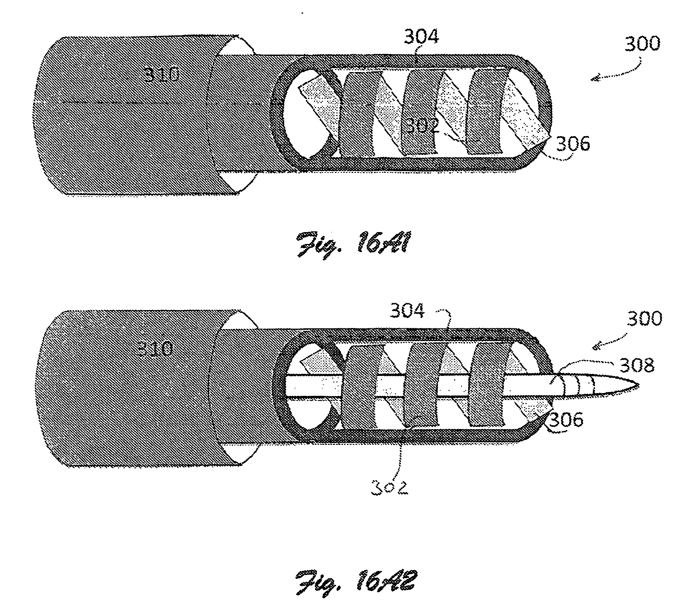

Figures 16A to 17C illustrate the use the embolectomy device, namely the endless wire with the spring-like helical member and the plurality of loops, in removing a clot.

As described above, endless wire 116 may be used with one of the specially dedicated catheter described above or any other standard catheter.

In figures 16A-17C the endless wire is illustrated with a more standard intravascular catheters. As described above, the wire is designed to draw clot into the distal end of such catheters. The user may select the catheter's diameter suitable for the target clot and the surrounding artery.

Figure 16A1 provides a partial, cutaway, side-view of the loops of the spring-like helical member (300) (of the endless wire) having a rotating helix (302) within a catheter body (304). The depicted helix (302) is shown as a ribbon; it may be constructed by cutting an appropriately sized hypotube into a helix or by winding a ribbon into the noted helix (302).

The width of the ribbon or thread may be constant or varying over the length of the helix. The helix (302) may have a constant pitch or a varying one. The helix (302) may be a single, double, or triple pitch.

The helix extends from the distal end proximally for at least a portion of the distance to a drive assembly that rotates the helix (302).

The helix (302) may have an extension (306) that, as the helix (302) is rotated, separates a target clot from an arterial wall and transports the clot into the mesh-like net as described above (not shown)and eventually through the catheter lumen (or).

An optional guide wire or core wire (308) (also denoted above as the corkscrew-like effecter 2002), that may be used to provide rotational or axial stability (see figure 16A2). The core wire (308) may be extended distally from the catheter body (304) into the body of a target embolism (or along the clot's periphery along the arterial wall) and, in many instances, provides a predictable center of rotation for the rotating helix.

The core wire can be used as a screw penetrating internally into the clot (unlike the loops of the endless wire described above which merely encircles the clot).

As described above, the clot can be initially encircled by the loops of the spring-like helical member, then by the net and only then be crossed by the core wire (308). Alternatively, the clot can be initially encircled by the net, then by the loops of the spring-like helical member, and only then be crossed by the core wire (308). Alternatively, the clot can be initially encircled simultaneously by both the net and by the loops of the spring-like helical member, and only then be crossed by the core wire (308).

Finally, Figures 16A1 and 16A2 illustrate an optional outer sheath (310) fitting loosely about catheter body (304) that allows the user to inject medicines or contrast agents at the clot site.

It should be emphasized that the rotatable spring-like helical effecter may be operated with or without the core wire (308).

A variation of this component involves affixing the rotatable spring-like helical effecter (302) to the core wire (308) at one or more sites and rotating the two together.

Typically, the catheter body (304) lumen will be attached to a vacuum source, although this is not required.

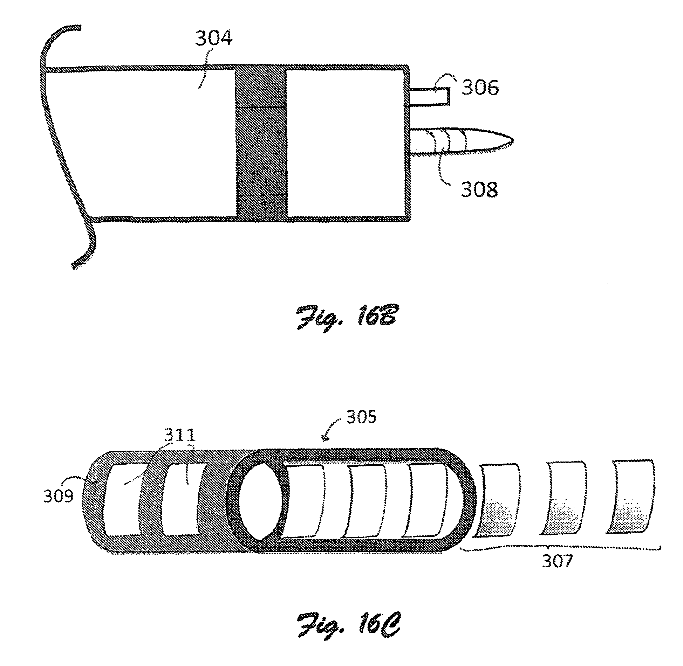

Figure 16B illustrates a side view of the extension (306) located on the spring-like helical member (of the endless wire)within a catheter body (304).

Figure 16C illustrates another embodiment (305) of the endless wire and namely, the spring-like helical member.

According to this embodiment, the depicted rotating helix assembly (305) comprises a distal ribbon-form helix (307) and a hollow more-proximal drive shaft (309). The drive shaft (309) is shown to have a number of flexibility slots (311) allowing the shaft (309) to smoothly flex during rotation of the rotating helix assembly (305) and to flex with greater bend angles. The ribbon-form helix (307) is attached to the drive shaft (309) by an appropriate joining method, e.g., welding, soldering, gluing, etc. The helix (307) and the drive shaft (309) may comprise the same materials of construction, e.g., metallic materials such as stainless steels or superelastic alloys such as nitinol and polymeric materials such as PEEK or a Nylon, or may be different.

The length of the ribbon-form helix (307) may be reasonably short, e.g., less than about 2-3 inches. Longer helix components may bind during rotation.

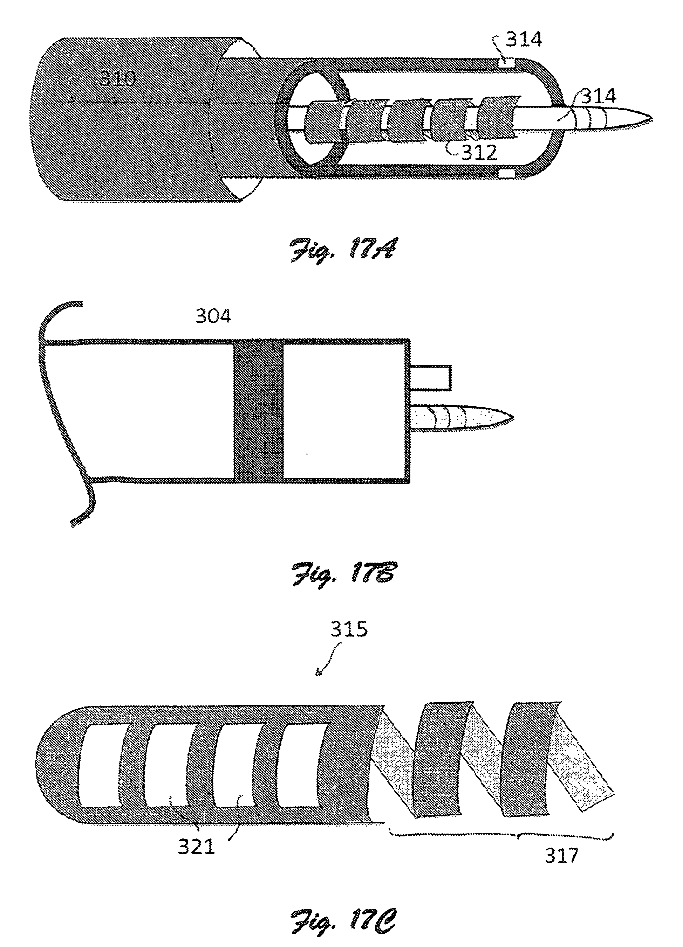

Figures 17A and 17B illustrate another embodiment (310) of the endless wire substantially similar to the variation shown in Figures 16A and 16B, with the exception of the shape of the rotating helix (312) which is a wire and that the wire-form rotating helix (312) is wrapped around a central core wire (314).

The wire-form rotating helix (312) and the central core wire (314) in this variation may rotate together, although this is not a requirement.

The wire-form rotating helix (312) may have a diameter such as seen with the ribbon-form rotating helix (302) shown in Figure 16A. Additionally the wire-form rotating helix (312) may be utilized without the central core wire (314).

The wire comprising the wire-form rotating helix (312) may have a circular cross-section or other cross sections suitable to the designer, e.g., oval or fin-like projections, to enhance carriage of the embolic fragments through the catheter body (306). The cross-section of that wire may be constant or vary as desired by the designer.

Figure 17C is a side view of another embodiment (315) of the endless wire. According to this embodiment, the endless wire (315) comprises a distal wire-form helix (317) and a hollow more-proximal drive shaft (319). The drive shaft (319) is also shown to have a number of flexibility slots (321). The wire-form helix (317) is attached to the drive shaft (319) by an appropriate joining method.

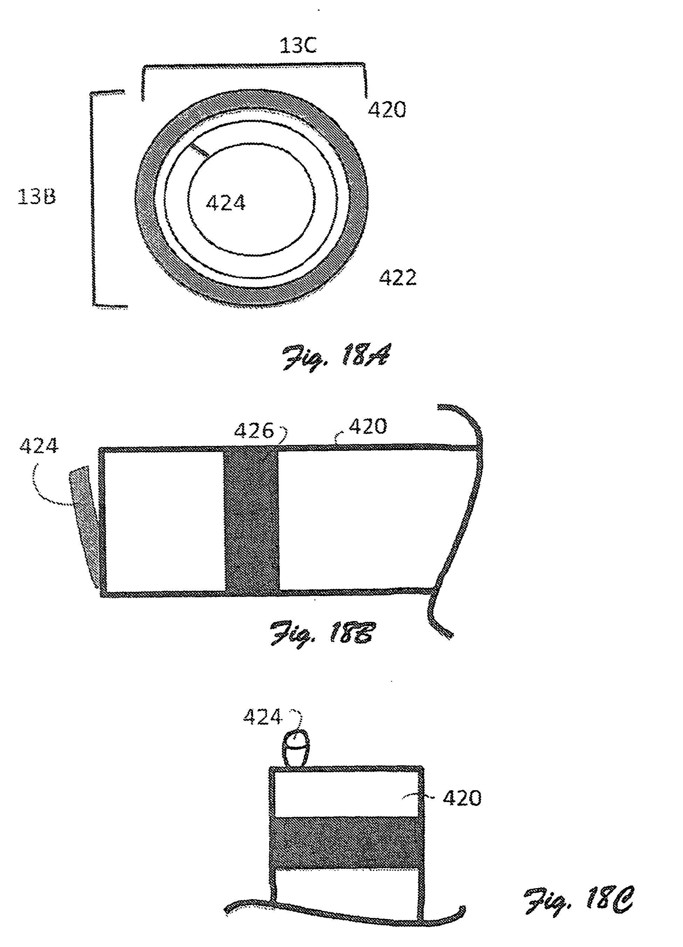

Figures 18A-18C show a variation of the rotating element in a catheter tip designed to extract a clot into the catheter in a substantially complete and whole form rather than macerating it.

Figure 18A provides an end view of the catheter (420) enclosing a rotating helical element (422) having an extended portion (424). Because the extended portion (424) has substantially no radial component, it may be used to separate a target clot from the artery wall, particularly when used with vacuum in the catheter lumen and with a guidewire.

Figure 18B shows a side-view of the catheter (420) and the tip or extension (424) of the rotating helical element. The distal most end of the extended portion (424) is blunt for the reasons of preventing damage to the intima of the artery and preventing penetration of the clot. The Figure also shows a radio-opaque band (426) allowing radiographic visualization of the position of that tip.

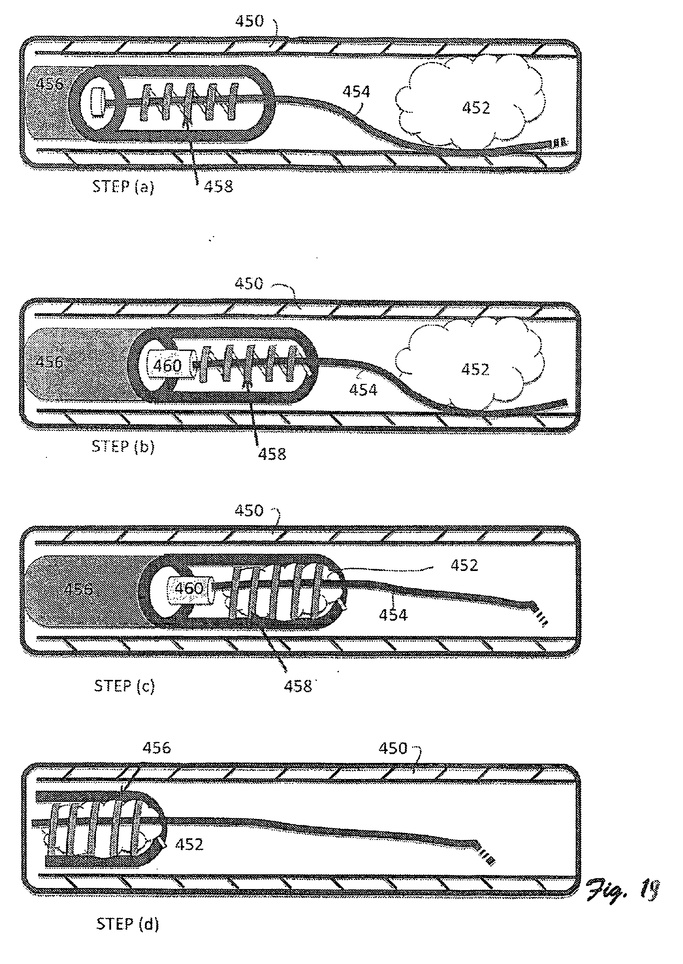

Figure 19 illustrates a method for using the endless wire, as described above for extracting a clot.

It is widely known that if a catheter is strongly attached to the clot and the clot is disengaged from the arterial wall, the clot may be safely and easily pulled from that vessel with minimum stress deflection to the wall. The pulling action helps reduce the friction between the clot and the vessel wall and can also help in removing the clot in one piece.

In step (a) a clot (452) in an artery (450) is shown. A guidewire (454) has been introduced past the clot (452). A catheter (456) containing the endless wire and the plurality of loop (458) approaches the clot (452).

The catheter (456) diameter has been chosen to approach the inner diameter of the artery (450).

In step (b), the distal end of the catheter (456) has contacted the clot (452). A vacuum may be applied to the catheter's (456) lumen to firmly attach the catheter (456) to the clot (452). Rotation of the helix (458) via the hollow drive shaft (460) begins. Such rotation encircles the clot from the out surface. It should be emphasized that the loops of the endless wire does not cross the clot. Such an action may lead to crumbling of the clot.

In step (c), the rotating helix (458) has disengaged the clot (452) from the arterial wall (450). Now the clot is being confined via the tubular mesh-like net (not shown in the figure). Finally clot (452) is being pulled into the catheter (456).

In step (d), the catheter (456) with the included clot (452) is being pulled and extracted from the artery (450).

It should be emphasized (as described above) that the clot can be encircled first by the net and then by the loops of the helical member (i.e., the helix); the clot can be first encircled by the the loops of the helical member (i.e., the helix) and then by the net; or simultaneously by the net and the loops.

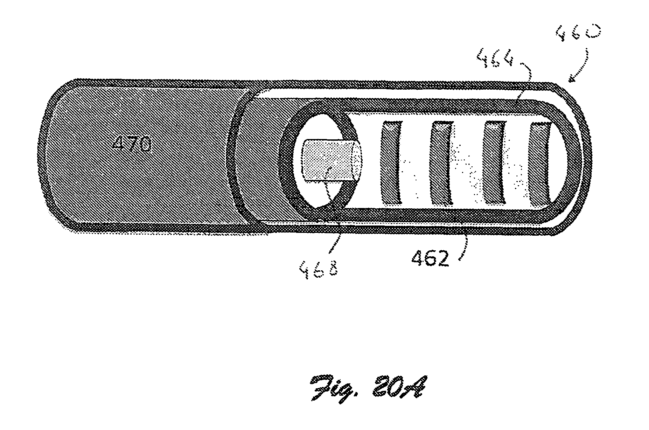

Figure 20A illustrates yet another embodiment of device (460) having both an expandable, rotatable, helical member (462) and a micro-catheter (464) having a distal section that is expandable upon reaching a target clot. This embodiment also includes a central core member, a guidewire (468), and an outer covering or sheath (470) allowing control of the expansion of the catheter (464) tip. The guidewire (468) may be withdrawn before beginning the grasping of the clot.

As noted above, the tip of the catheter (464) and the rotatable member (462) are expanded to a size approximating the inner diameter of the vessel in the region of the target clot to aid in the separation of the clot from the vessel wall. Such separation reduces the force needed to extract the clot from the vessel and also enhances the potential that the clot may be extracted from the vessel as a single, undivided mass.

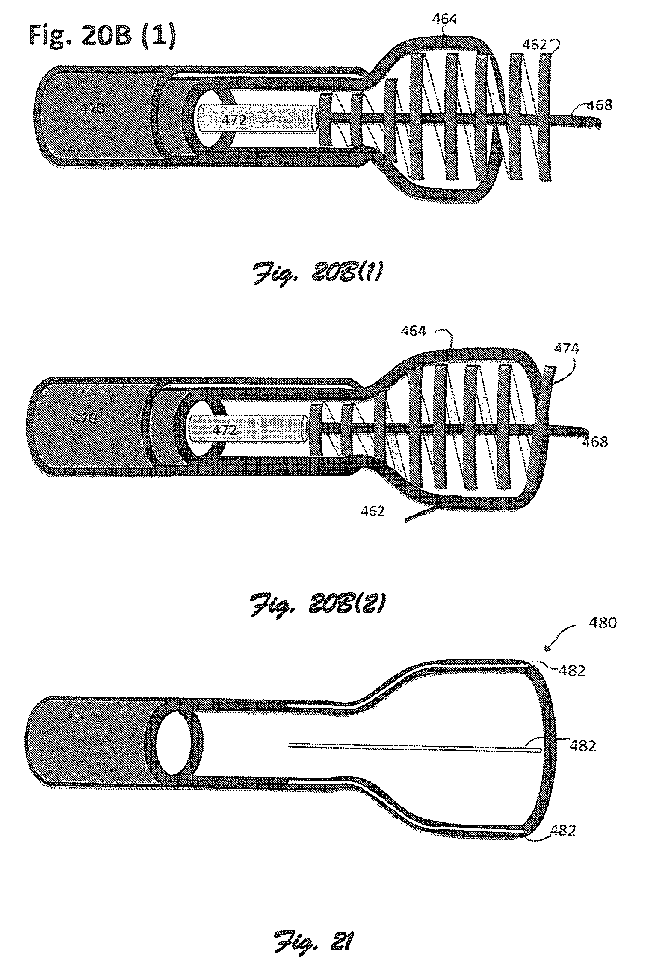

Figures 20B(1) and 20B(2) show two variations of the device (460) after expansion of the catheter (464) and the rotatable member (462).

The more-proximal driving member (472) used to turn the rotatable member (462) is also visible in the Figures.

Figure 20B(1) shows the variation in which the loops(462) of the spring-like helical member of the endless wire extend distally from the micro-catheter (464) when extracting the target clot. In operation, the rotating member (462) is turned using the driving member (472) and at least partially envelopes the target clot and subsequently or simultaneously enclosed by the mesh-like net and eventually drawn into the catheter (464) for extraction from the blood vessel.

On the other hand, Figure 20B(2) shows a variation in which the loops(462) of the spring-like helical member is maintained substantially within the catheter (464) tip. In this variation, the clot is drawn into the catheter (464) tip rather than being pulled in later. As the rotating member (462) is turned, it encircles the clot and then the clot is being enclosed by the net (as mention above this operation mode is optionally).

It should be emphasized that both the loops of the spring-like helix member and the mesh-like net can be characterized by a varied dimensions. Furthermore, the spring-like helix member may have a constant and/or a varying pitch.

Figure 21 illustrates another embodiment of an expandable catheter tip (480) having preshaped, longitudinal ribs (482) that, upon withdrawal of the control sheath (470 in Figures 16A, 16B(1), and 16B(2)) form an expanded region in the catheter tip (480). Although the supporting ribs (482) may be springy, e.g., comprising an elastic material such as nitinol or the like, they may also be malleable and formed in-situ perhaps by the expandable rotatable member.

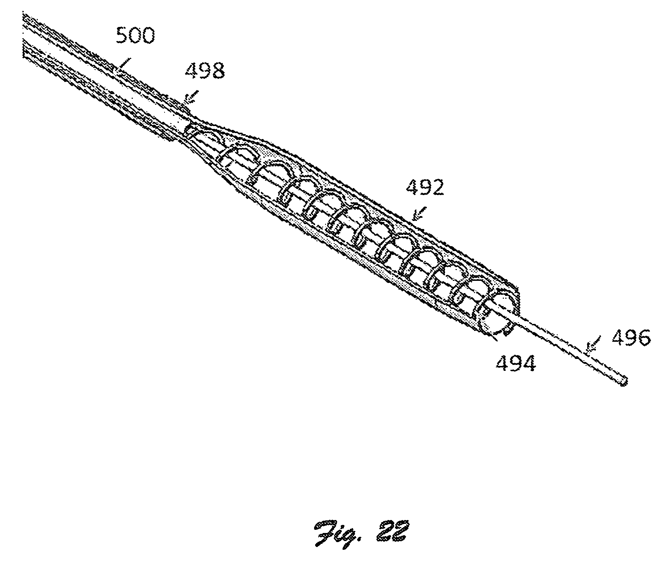

Figure 22 provides a perspective, partial cutaway view of another variation of the device (490) shown in Figures 20A and 20B(2). The variation (490) includes the expanded microcatheter (492), expanded rotatable member (494), guidewire (496), and control sheath (498). The driving member (500) for rotating the rotatable member (494) is also visible.

In this variation and in the other variations of the rotating member shown here, the cross-section of the wire itself may have a variety of cross-sections, e.g., round, oval, square, rectangular, triangular, hollow, etc. and other irregular shapes.

If hollow, the rotating member may be used to advance contrast material to its distal tip. The wire or ribbon making up the rotating member may be at least partially wound with a smaller wire to accomplish either or both of the goals of providing radio-opacity to at least a part of the rotating member -- by applying a radio-opaque wire comprising, e.g., platinum or goldor to enhance the resistance of the member to kinking.

It should be emphasized that said embodiment (490) additionally comprises a tubular net (not shown in the figure) into which the clot is inserted. As described above, the net and/or the helix may have varying dimensions.

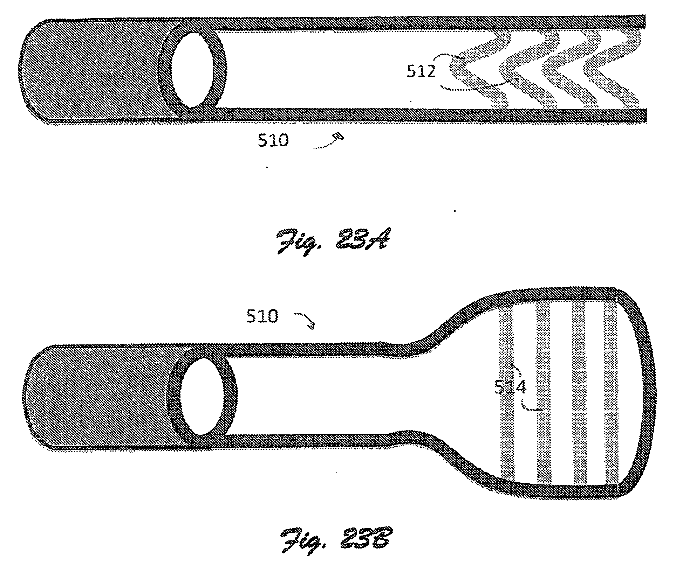

Figures 23A and 23B illustrate, respectively, a collapsed catheter tip (510) having individual undulating rings (512 in Figure 23A) that expand, upon release, to form circular rings (514 in Figure 23B). Again, the rings (512, 514) may be formed of a springy material having a preformed shape or of a malleable material that is formed into rings in situ.

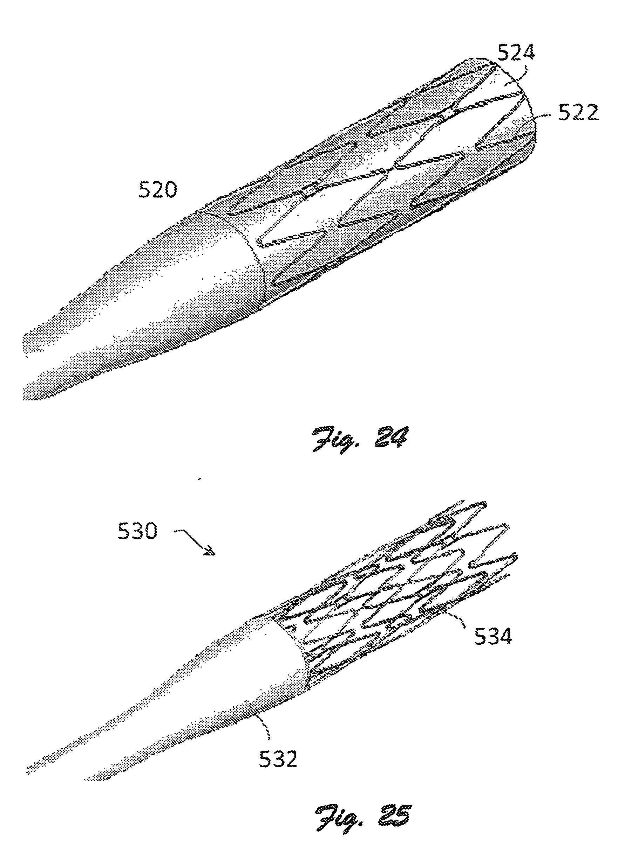

Figure 24 illustrates another variation of a catheter tip (520) in which the structure used to expand and maintain the shape of the expanded catheter tip (520) comprises a stent-like structure (522). In this variation, the stent-like structure (522) is included within (or attached to) the catheter wall (524). The strength of the structure (522) need not, of course, be of that needed in stent service, since the strength needed to hold the catheter open is much less than that needed to maintain the patency of an artery. Said another way: the structure (522) is fully retractable.

Figure 25 shows still another variation of a catheter tip assembly (530) in which the structure used to expand and maintain the shape of the expanded catheter up (532) comprises a stent-like structure (534). In this variation, the stent-like structure (534) is bare but attached to the body of the catheter tip (530). Again, the structure (534) may be self-expanding or expanded using a balloon or the like. The structure may be coated with a lubricious coating, e.g., such as a polymer, to increase the ease with which the structure (534) moves between the embolism and the vessel wall during introduction.

The stent-like structures (522, 534) may be of any convenient form such as shown in the Figures or may be of another similar shape having the functions described above. Braided wires or ribbons or laser cut tubes are also appropriate.

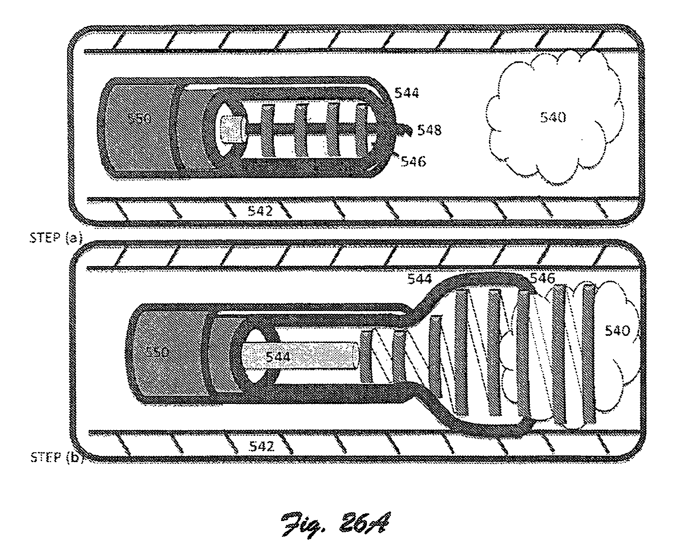

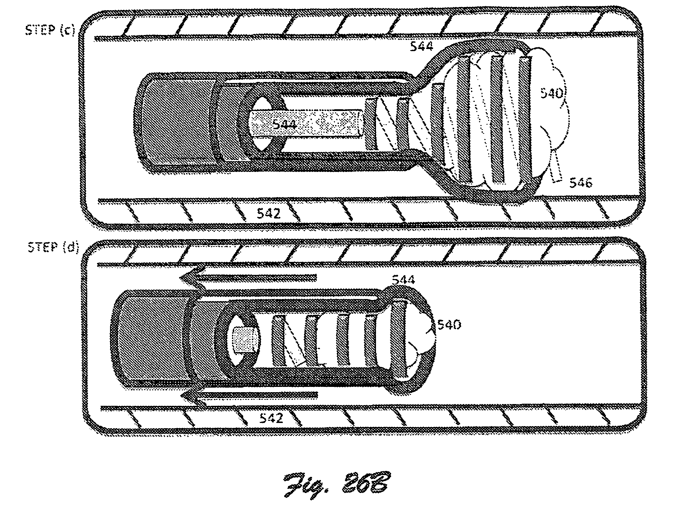

Figure 26 illustrates a method for utilizing the embolectomy device.

In step (a) of Figure 26 a clot (540) lodged in artery (542) is illustrated. The microcatheter (544) having an expandable distal end, an endless wire (not shown) coupled to a spring-like helical member having a plurality of loops (546), and a guidewire (548). A retractable sheath (550) for controlling and initiating expansion of, and for controlling the retraction of, the microcatheter (544) and the rotatable helical member (546), is also shown.

Step (b) illustrates the extension of the microcatheter (544) from the sheath (550) and the selfexpansion of the distal end of the microcatheter (544) and the rotating helix member (546). The expanded distal end of the microcatheter (544) has contacted the clot (540) and the rotation of the helical member (546) has provided progression of that helical member (546) partially around the exterior of the clot (540).

As a practical matter, there are several ways that the helical member (546) may cooperate with the microcatheter (544) in enveloping the clot. First, the rotatable member (546) may move axially along the clot (540) and followed by a subsequent axial movement of the catheter (544). Second, the rotatable member (546) may be rotated, enveloping the clot (540), while the catheter (544) is held stationary, or at least not rotated. Third, the rotatable member (546) is rotated, enveloping the clot (540), and the catheter (544) is simultaneously rotated.

Step (c) shows the retraction of the clot (540) into the catheter's (544) distal region or tip. In the effectuation of steps (a) and (b), the clot (540) has been separated from the wall of the artery (542) lessening the force needed to remove the clot (540). The catheter (544) is then withdrawn extracting the clot from the artery (542) and restoring blood flow to the artery (542).

Alternatively, the clot is first encircle by said rotatable member (546) and then inserted into a tubular mesh-like net (not shown) which is then extracted from the artery (542). Yet alternatively, the clot is simultaneously encircle by said rotatable member (546) and the tubular mesh-tike net; only then extracted from the body.

In the foregoing description, embodiments of the invention, including preferred embodiments, have been presented for the purpose of illustration and description. They are not intended to be exhaustive or to limit the invention to the precise form disclosed. Obvious modifications or variations are possible in light of the above teachings. The embodiments were chosen and described to provide the best illustration of the principals of the invention and its practical application, and to enable one of ordinary skill in the art to utilize the invention in various embodiments and with various modifications as are suited to the particular use contemplated. All such modifications and variations are within the scope of the invention as determined by the appended claims when interpreted in accordance with the breadth they are fairly, legally, and equitably entitled.

## Claims

1. A device for extracting a clot. (1010), from a blood vessel (1020) in a non fragmented manner, comprising
an endless wire (116) coupled to a spring-like helical member (2101) having a plurality of loops (2201); said loops are positioned at an angle A relatively to the main longitudinal axis of said blood vessel (1020); and, are adapted to radially encircle at least a portion of the outer circumference of said clot: and,
a tubular mesh-tike net (1100) for both enveloping said helical member and enclosing said clot therein;
wherein when at least a portion of said clot is contemporaneously confined within said loops and said mesh-like net, the devise is adapted to exert radial compression forces and longitudinal shearing forces on said clot such that the tendency of said clot to fragment is mitigated **characterized by** a corkscrew-like effecter (118), having a coil-like (2002) distal end, adapted to penetrate and cross said clot (1010).

2. The embolectomy device of claim 1, wherein said spring-like helical member (2101) and/or said mesh-like net (1100) comprising means for applying vibration on said clot.

3. The embolectomy device of claim 1, wherein said mesh-like net (1100) is made of material selected from a group consisting of woven fabrics, non-woven fabrics, gauze-like fabrics, materials having multiple openings, and fiuid-permeable membranes or any combination thereof.

4. The embolectomy device of claim 1, wherein said mesh-like net (1100) additionally comprising materials selected from a group consisting of woven fabrics, non-woven fabrics, gauze-like fabrics, materials having multiple openings, and fluid-permeable membranes or any combination thereof.

5. The embolectomy device of claims 1, wherein said spring-like helical member (2101) is coupled to said mesh-like net (1100).

6. The embolectomy device of claim 1, wherein said distal portion of said mesh-like net (1100) is Adapted to switch from an expended-configuration (1013) to a restricted-configuration (1014); said expended-configuration (1013) is operative to allow insertion of at least a portion of a target clot (1010) in to said mesh-like net's distal portion: and said restricted configuration, (1014) is operative to retain said clot (1010) within said mesh-like net (1100).

7. The embolectomy device of claim 6, wherein said distal portion of said mesh-like net (1100) is operative to self-expand from its expended-configuration (1013) to its restricted-configuration (1014).

8. The embolectomy device of claim 1, wherein said mesh-like net (1100) is an elongated sleeve comprising a restricting sphincter located at the distal end of said sleeve, adapted to retain at least a portion of the target clot within said mesh-like net.

9. The embolectomy device of claim 1, wherein said helical member (2101) is **characterized by** a cross-sectional area selected from a group consisting of circular, oval, round, square, rectangular, triangular, regular or irregular shapes, or any combination thereof.

10. The embolectomy device of claim 1, Additionally comprising a motor adapted to rotate said helical member (2101).

11. The embolectomy device of claim 1, wherein said helical member additionally comprising protrusions for assisting in the separation of said clot from said vasculature.

12. The embolectomy device of claim 1, additionally comprising vibration means applied on said clot.

13. The embolectomy device of claim 1, wherein said distal effecter is incorporated or otherwise connected with said proximal effecter to form a single effecter.

14. The embolectomy device of claim 1, wherein said angle A ranges from about 0 degrees to about 180 degrees.

15. The embolectomy device of claim 1 , additionally comprising aspiration means.

16. The embolectomy device of claim 1, wherein at least one of said loops (2201) and/or said mesh-like net (1100) is coated with lubricious polymeric material selected from hydrophilic polymer material.

17. The embolectomy device of claim 1, wherein said spring-like helical member (2101) is **characterized by** single, double, or triple pitch.

18. The embolectomy device of claim 1, wherein the cross section of at least one of said loops and/or said mesh-like net varies.

## Patentansprüche

1. Eine Vorrichtung zur Extraktion eines Gerinnsels (1010) von einem Blutgefäß (1020) in einer nichtfragmentierten Weise, umfassend:
einen endlosen Draht (116), welcher an ein federähnliches spiralförmiges Teil (2101) gekoppelt ist, welches eine Vielzahl von Schleifen (2201) umfasst; wobei die Schleifen relativ zu der longitudinalen Hauptachse des Blutgefäßes (1020) in einem Winkel A angeordnet sind; und dazu ausgebildet sind, zumindest einen Teil des äußereren Umfangs des Gerinnsels radial zu umfassen; und
ein röhrenförmiges, gitterartiges Netz (1100) zum Einhüllen des spiralförmigen Teils und zum Einhüllen des Gerinnsels darin;
wobei wenn zumindest ein Teil des Gerinnsels vorübergehend in den Schleifen und dem gitterartigen Netz eingeschlossen ist, die Vorrichtung dazu ausgebildet ist, radiale Kompressionskräfte und longitudinale Scherkräfte auf das Gerinnsel auszuüben, so dass die Tendenz des Gerinnsels zur Fragmentierung gelindert wird; **gekennzeichnet durch**
einen korkenzieherartigen Effektor (118), welcher ein spulenartiges (2002), distales Ende aufweist, welches dazu ausgebildet ist, in das Gerinnsel (1010) einzudringen und es zu durchqueren.

2. Die Embolektomievorrichtung gemäß Anspruch 1, wobei das federartige, spiralförmige Teil (2101) und/oder das gitterartige Netz (1100) Mittel zum Anwenden von Vibrationen auf das Gerinnsel umfassen.

3. Die Embolektomievorrichtung gemäß Anspruch 1, wobei das gitterartige Netz (1100) aus einem Material der folgenden Gruppe gebildet ist: gewebte Stoffe, nichtgewebte Stoffe, florartige Stoffe, Materialien mit mehreren Öffnungen und fluiddurchlässige Membranen oder jegliche Kombination dieser.

4. Die Embolektomievorrichtung gemäß Anspruch 1, wobei das gitterartige Netz (1100) zusätzlich Materialien der folgenden Gruppe umfasst: gewebte Stoffe, nichtgewebte Stoffe, florartige Stoffe, Materialien mit mehreren Öffnungen und fluiddurchlässige Membranen oder jegliche Kombination dieser.

5. Die Embolektomievorrichtung gemäß Anspruch 1, wobei das federartige, spiralförmige Teil (2101) mit dem gitterartigen Netz (1100) verbunden ist.

6. Die Embolektomievorrichtung gemäß Anspruch 1, wobei der distale Bereich des gitterartigen Netzes (1100) ausgebildet ist zum Schalten von einer ausgedehnten Konfiguration (1013) zu einer beschränkten Konfiguration (1014), wobei die ausgedehnte Konfiguration (1013) das Einfügen mindestens eines Bereichs des Zielgerinnsels (1010) in das distale Ende des gitterartige Netzes ermöglicht; und in der beschränkten Konfiguration (1014) das Halten des Gerinnsels (1010) in dem gitterartigen Netz (1100) ermöglicht.

7. Die Embolektomievorrichtung gemäß Anspruch 6, wobei der distale Bereich des gitterartigen Netzes (1100) zum Selbstexpandieren von seiner ausgedehnten Konfiguration (1013) zu seiner beschränkten Konfiguration (1014) ausgebildet ist.

8. Die Embolektomievorrichtung gemäß Anspruch 1, wobei das gitterartige Netz (1100) eine längliche Hülse ist, welche einen beschränkenden Schließmuskel besitzt, der am distalen Ende der Hülse angeordnet ist und dazu ausgebildet ist, zumindest einen Bereich des Zielgerinnsels innerhalb des gitterartigen Netzes zu halten.

9. Die Embolektomievorrichtung gemäß Anspruch 1, wobei das spiralförmige Teil (2101) durch einen Querschnittsbereich **gekennzeichnet** ist, welcher aus der folgenden Gruppe ausgewählt ist: kreisförmig, oval, rund, quadratisch, rechteckig, dreieckig, regelmäßige oder unregelmäßige Formen oder Kombinationen dieser.

10. Die Embolektomievorrichtung gemäß Anspruch 1, weiterhin umfassend einen Motor zur Rotation des spiralförmigen Teils (2101).

11. Die Embolektomievorrichtung gemäß Anspruch 1, wobei das spiralförmige Teil weiterhin Vorsprünge zum Bereitstellen einer Separierung des Gerinnsels von dem Gefäß umfasst.

12. Die Embolektomievorrichtung gemäß Anspruch 1, weiterhin umfassend Vibrationsmittel, welche auf das Gerinnsel angewendet werden.

13. Die Embolektomievorrichtung gemäß Anspruch 1, wobei der distale Effektor zum Bilden eines einzigen Effektors in den proximalen Effektor integriert ist oder anders mit diesem verbunden ist.

14. Die Embolektomievorrichtung gemäß Anspruch 1, wobei der Winkel A zwischen 0 Grad und ungefähr 180 Grad liegt.

15. Die Embolektomievorrichtung gemäß Anspruch 1, weiterhin umfassend Mittel zum Absaugen.

16. Die Embolektomievorrichtung gemäß Anspruch 1, wobei mindestens eine der Schleifen (2201) und/oder das gitterartige Netz (1100) mit einem schmierenden, polymeren Material beschichtet ist, welches von hydrophilem, polymerem Material ausgewählt ist.

17. Die Embolektomievorrichtung gemäß Anspruch 1, wobei das federartige, spiralförmige Teil (2101) durch einen einfachen, doppelten oder dreifachen Gewindegang **gekennzeichnet** ist.

18. Die Embolektomievorrichtung gemäß Anspruch 1, wobei der Querschnitt von mindestens einer der Schleifen und/oder dem gitterartigen Netz variiert.

## Revendications

1. Dispositif pour extraire un caillot (1010) d'un vaisseau sanguin (1020) d'une manière non fragmentée, comprenant :
un fil métallique sans fin (116) couplé à un membre hélicoïdal analogue à un ressort (2101) possédant plusieurs boucles (2201), lesdites boucles étant positionnées en formant un angle A par rapport à l'axe longitudinal principal dudit vaisseau sanguin (1020) et étant conçues pour encercler en direction radiale au moins une portion de la circonférence externe dudit caillot ; et
un filet tubulaire analogue à un treillis (1100) pour à la fois envelopper ledit membre hélicoïdal et y renfermer ledit caillot sanguin ;
dans lequel, lorsqu'au moins une portion dudit caillot est confinée en même temps au sein desdites boucles et dudit filet analogue à un treillis, le dispositif est conçu pour exercer des forces de compression en direction radiale et des forces de cisaillement en direction longitudinale sur ledit caillot, de telle sorte que l'on réduit la tendance manifestée par le caillot à se fragmenter ;
**caractérisé par** un effecteur analogue à un tire-bouchon (118) possédant une extrémité distale (2002) de forme hélicoïdale conçue pour pénétrer dans ledit caillot (1010) et pour le traverser.

2. Dispositif d'embolectomie selon la revendication 1, dans lequel ledit membre hélicoïdal (2101) analogue à un ressort et/ou ledit filet analogue à un treillis (1100) comprennent un moyen pour appliquer des vibrations sur ledit caillot.

3. Dispositif d'embolectomie selon la revendication 1, dans lequel ledit filet analogue à un treillis (1100) est réalisé en une matière choisie parmi le groupe constitué par des étoffes tissées, des non-tissés, des tissus de la texture de la gaze, des matières possédant de multiples ouvertures et des membranes perméables aux fluides, ou l'une quelconque de leurs combinaisons.

4. Dispositif d'embolectomie selon la revendication 1, dans lequel ledit filet analogue à un treillis (1100) comprend en outre des matières choisies parmi le groupe constitué par des étoffes tissées, des non-tissés, des tissus de la texture de la gaze, des matières possédant de multiples ouvertures et des membranes perméables aux fluides, ou l'une quelconque de leurs combinaisons.

5. Dispositif d'embolectomie selon la revendication 1, dans lequel ledit membre hélicoïdal (2101) analogue à un ressort est couplé audit filet analogue à un treillis (1100) .

6. Dispositif d'embolectomie selon la revendication 1, dans lequel ladite portion distale dudit filet analogue à un treillis (1100) est conçue pour passer par commutation d'une configuration à l'état déployé (1013) à une configuration à l'état restreint (1014), ladite configuration à l'état déployé (1013) étant opérationnelle pour permettre l'insertion d'au moins une portion d'un caillot cible (1010) dans ladite portion distale dudit filet analogue à un treillis, et ladite configuration à l'état restreint (1014) étant opérationnelle pour retenir ledit caillot (1010) au sein dudit filet analogue à un treillis (1100).

7. Dispositif d'embolectomie selon la revendication 6, dans lequel ladite portion distale dudit filet analogue à un treillis (1100) est opérationnelle pour se déployer d'elle-même en passant de sa configuration à l'état déployé (1013) à sa configuration à l'état restreint (1014) .

8. Dispositif d'embolectomie selon la revendication 1, dans lequel ledit filet analogue à un treillis (1100) est un manchon allongé comprenant un sphincter de constriction qui est disposé à l'extrémité distale dudit manchon, conçu pour retenir au moins une portion du caillot cible au sein dudit filet analogue à un treillis.

9. Dispositif d'embolectomie selon la revendication 1, dans lequel ledit membre hélicoïdal (2101) est **caractérisé par** une aire de section de passage, choisie parmi le groupe constitué par une configuration circulaire, ovale, ronde, carrée, rectangulaire, triangulaire, régulière ou irrégulière, ou l'une quelconque de leurs combinaisons.

10. Dispositif d'embolectomie selon la revendication 1, comprenant en outre un moteur conçu pour faire tourner ledit membre hélicoïdal (2101).

11. Dispositif d'embolectomie selon la revendication 1, dans lequel ledit membre hélicoïdal comprend en outre des saillies pour faciliter la séparation dudit caillot par rapport audit système vasculaire.

12. Dispositif d'embolectomie selon la revendication 1, comprenant en outre un moyen vibratoire appliqué sur ledit caillot.

13. Dispositif d'embolectomie selon la revendication 1, dans lequel ledit effecteur distal est incorporé dans ledit effecteur proximal ou y est relié d'une autre manière afin d'obtenir un effecteur unique.

14. Dispositif d'embolectomie selon la revendication 1, dans lequel ledit angle A s'étend entre environ 0° et environ 180°.

15. Dispositif d'embolectomie selon la revendication 1, comprenant en outre un moyen d'aspiration.

16. Dispositif d'embolectomie selon la revendication 1, dans lequel au moins un membre choisi parmi lesdites boucles (2201) et/ou ledit filet analogue à un treillis (1100) est enduit d'une matière polymère possédant un pouvoir lubrifiant, choisie parmi des matières polymères hydrophiles.

17. Dispositif d'embolectomie selon la revendication 1, dans lequel ledit membre hélicoïdal analogue à un ressort (2101) est **caractérisé par** un pas unique, un double pas ou un triple pas.

18. Dispositif d'embolectomie selon la revendication 1, dans lequel la section transversale d'au moins un membre choisi parmi lesdites boucles et/ou ledit filet analogue à un treillis varie.
